# EUROPEAN PATENT SPECIFICATION

(11) **EP 3 810 162 B1**
(45) Date of publication and mention of the grant of the patent: **20.11.2024**
(21) Application number: 18755287.2
(22) Date of filing: 12.07.2018
(51) Int. Cl.: A61K 35/76, C12N 7/00, A61P 31/04

(54) **ANTIMICROBIAL COMPOSITION AGAINST GASTROINTESTINAL INFECTIONS AND THE USE OF THEREOF**
ANTIMIKROBIELLE ZUSAMMENSETZUNG GEGEN GASTROINTESTINALE INFEKTIONEN UND VERWENDUNG DAVON
COMPOSITION ANTIMICROBIENNE CONTRE LES INFECTIONS GASTRO-INTESTINALES ET SON UTILISATION

(30) Priority: 02.05.2018 GE AP2018014772
(43) Date of publication of application: 28.04.2021
(73) Proprietor: JSC "BIOCHIMPHARM", Tbilisi 0160 (GE)
(72) Inventor: GOLIJASHVILI, Aleksander, Mtskheta, 3311 (GE); GOLIJASHVILI, Rati, Tbilisi, 0171 (GE); DZULIASHVILI, Mariam, Tbilisi, 0186 (GE); PAPUKASHVILI, Irina, Tbilisi, 0186 (GE)
(74) Representative: Kretschmann, Dennis
(86) International application number: PCT/GE2018/000002
(87) International publication number: WO 2019/211634

(56) References cited:
- EP-A1- 2 781 220
- WO-A1-2009/075884
- KR-A- 20130 021 677
- RU-C1- 2 412 243
- US-A1- 2013 336 932
- CISEK AGATA ANNA ET AL: "Phage Therapy in Bacterial Infections Treatment: One Hundred Years After the Discovery of Bacteriophages", CURRENT MICROBIOLOGY, SPRINGER, BOSTON, vol. 74, no. 2, 28 November 2016 (2016-11-28), pages 277 - 283, XP036136594, ISSN: 0343-8651, [retrieved on 20161128], DOI: 10.1007/S00284-016-1166-X

## Description

### Field of the Invention

The present invention belongs to pharmaceutical industry, biotechnology, medicine, veterinary medicine, environmental protection and relates to an antimicrobial composition against gastrointestinal infections, preparations made on its basis, its constituent bacteriophage strains and the use thereof.

### Prior Art

Treatment and prevention of bacterial infections are a global problem of modern medicine.

Bacterial infections are not only an important public health issue, but also they are a significant and increasing problem for veterinary medicine, environmental safety and economics. According to the data of World Health Organization (WHO), European Food Safety Authority (EFSA) and European Centre for Disease Prevention and Control (ECDC), public health risks related to anthroponotic bacterial diseases, including salmonellosis, escherichiosis, shigellosis, *Staphylococcus, Streptococcus, Pseudomonas* infections - are increasing each year. Animals or fowls, affected with bacterial infections, may become a source of infection for humans; animal and poultry products used as food - such as meat, egg - contaminated with microorganisms (*Salmonelleae, Shigellae,* enteropathogenic *Escherichia coli, Proteae,* enterotoxigenic strains of *Staphylococcus* spp. and others), may cause toxicoinfections, intoxications and infectious diseases as well. Furthermore, the sources of infection during the secondary contamination of animal/poultry meat and products thereof, are environmental objects (soil, water, transport etc.), as well as infected people or asymptomatic carriers.

According to the experts of European Food Safety Authority (EFSA), economic burden of anthroponotic diseases may exceed 3 billion euros per year in Europe. Continuous increase in anthroponotic bacterial infections in various countries of the world, growth in the number of bacterial agents (their serovars) found in humans, animals and poultry, considerable microbial contamination of food products, environmental objects and economic burden therefrom, make these infections not only medical, but also ecological, veterinary and social problem.

According to WHO, bacterial infections are distinguished by severe disease progression and difficulty in their ellimination. One of the reasons, leading to these problems, is a large number of pathogenic microorganisms (their serotypes). The most noteworthy issue is increasing antibiotic resistance of infection-causing bacterial agents and global spread of their multidrug-resistant strains. One more challenge, facing physicians, is bacterial carriage too, not only in humans, but also in animals and poultry; for instance, carriage prevalence of *Salmonelleae* is up to 50%.

Generally, antibiotic resistant microorganisms play a key role in the development of bacterial purulent-inflammatory and intestinal infections. Throughout the years, widespread consumption of antibiotics without clinical urgency and strict monitoring for treatment of bacterial infections conditioned emergence and ubiquity of antibiotic-resistant bacterial strains, which is a logical consequence of evolutionary process of microbial development. Due to irrational consumption of antibiotics, we may appear as vulnerable to infections, as we were before the discovery of penicillin, - as stated by World Health Organization. Considering multidrug-resistance of bacterial pathogens, contraindications and complications of antibiotics (allergic reactions, toxic, immunosuppressive, embryotoxic, teratogenic effects, disruption of normal gut microflora - dysbiotic changes and etc.), the issue of treatment and prevention of bacterial infections is still of great importance in practical medicine, needs new methods of approach and development of optimal treatment plans.

Strictly specific, harmless, safe, effective biopreparations - bacteriophages, having the ability to intentionally eliminate conditionally-pathogenic microorganisms - one of the alternatives to antibiotics - are recognized as one of the most biological preparations for treatment and prevention of purulent-inflammatory and intestinal infections (used in humans, animals and poultry, as well as in environmental sanitation).

Bacteriophages or simply phages are bacterial viruses, which cause specific lysis of microorganisms, conditioning bacterial infections - they selectively disrupt bacterial cells. Bacteriophage adsorbs to the membrane of a homologous bacterial cell, disrupts its integrity, penetrates inside, multiplies and causes its lysis, along with releasing of a new population of phage progeny. ( 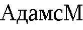 (1961) 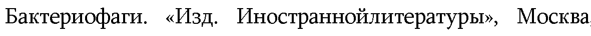 ; 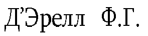 (1935) 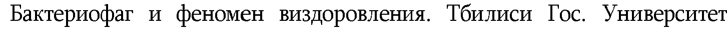 ; 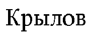 B.H. (2001) 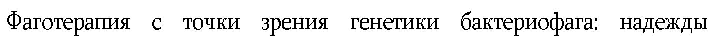 , 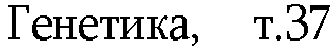 , Nº7, c.869-887; 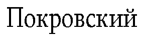 B.H., 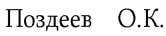 , 1998, 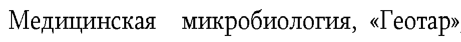 , 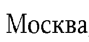 ; Abedon S.T. et al., (2001) Bacteriophage latent-period evolution as a response to resource availability. Applied and Environmental Microbiology, vol. 67, N9 pp. 4233-4241). Re-derived phage progeny continues infecting new microbial cells until infection liquidation - exactly the above-mentioned process is a basis of the successful phage therapy and prophylaxis. Nowadays, up to 4000 bacteriophage isolates, specific to approximately 100 bacterial genera are studied. (Ackerman H.W. and BertiaumeLaurenr., Atlas of viruses diagrams, CRC Press, Boca Ration, 1995, New York, London, Tokyo).

Phage therapy was developed shortly after the discovery of bacteriophages (1917. Felix d'Herelle). In the 1920s, D'Herelle worked out monocomponent dysentery-phage, as well as polycomponent intesti- and pyobacteriophage preparations for treatment and prevention of intestinal and purulent-surgical infections. In 1923 G.Eliava together with D'Herelle founded Institute of Bacteriology, Microbiology and Virology (IBMV) in Georgia. A lot of phages, specific to various bacterial pathogens, were isolated and studied in Georgia. The similar activities also took place in Russia, France and Poland. In the 1940s, shortly after beginning of the antibiotic era, phage therapy was forgotten in Western Europe. However, after the 1980s, increase in life threatening, antibiotic resistant bacterial infections and spread of them, as well as the possibility of deriving their new forms, rekindled interest in phage preparation consumption (as an alternative remedy to antibiotics) for treatment and prevention of bacterial infections. (Wei H., Bacteriophages, revitalized after 100 years in the shadow of antibiotics. Virol Sin., 2015, 30(1):1-2. doi: 10.1007/s12250-014-3562-y ; Wittebole X, De Roock S, Opal SM., A historical overview of bacteriophage therapy as an alternative to antibiotics for the treatment of bacterial pathogens, Virulence, 2014, 1;5(1):226-35. doi: 10.4161/viru.25991). Nowadays, too much attention is focused on this field in the West - phage therapeutic companies are founded, and bacteriophages are actively promoted and advertised. Scientific-clinical researches, which have been conducted for many years, reveal a number of significant factors, determining phage advantages over antibiotics and other chemical antibacterial preparations. These advantages are as follows:
1. Bacteriophages are natural antagonists of bacteria: they reveal high therapeutic potential (high lytic activity up to 80-90%) against pathogenic and conditionally pathogenic microorganisms;
2. Phages have the ability to self-replicate, lyse the antibiotic-resistant bacteria, reproduce rapidly, adapt and restrict the development of bacterial resistance to bacteriophages;
3. Bacteriophages multiply only in the presence of susceptible host bacteria; after lysis of the last microbial cell, phages are fully excreted from the body;
4. Phage ability to penetrate into different liquids, tissues and organs of the body, is extremely high (bacteriophage emerges in the blood in 1 hour, and at the site of infection - the first several hours after administration of phage preparation); - Investigation of phage pharmacokinetics and pharmacodynamics proved that bacteriophages, administered inside the body by any route (*per os*, topically or into the body cavities) - are absorbed quickly into the bodily fluids - blood and lymph - (1-1,5 h) and reach the focus of infection through the blood in the first several hours. They multiply into the homologous, phage-sensitive microbial pathogens and cause their lysis, while releasing new population of phage progeny. Bacteriophages are excreted from the body basically through the kidneys, causing urinary tract sanitation, and partially through the gastrointestinal tract;
5. Phage preparations are harmless, strictly specific; they cause neither side effects and complications, nor dysbiotic events; conversely, bacteriophages are used for their correction, are not characterized by cytotoxic effects and do not have an impact on the body metabolism;
6. Bacteriophage stimulates specific and non-specific factors of the immune system;
7. Rational combination of therapeutic phages with other antibacterial preparations, despite their class, is always synergistic. Phages are fully compatible with other drugs, including antibiotics; Application of phage preparations in combination with antibiotics reinforces the efficacy of the latter.
8. There is no correlation between phage-sensitivity and antibiotic-resistance;
9. Phage preparations are effectively used for prevention of bacterial infections;
10. Phage preparations have relatively low cost price and short timeline of development compared to antibiotics.
11. Bacteriophage production is an environment-friendly process.

Thus, consumption of therapeutic phage preparations - alternative to antibiotics - is of great importance for practical medicine to treat and prevent various bacterial infections. Over the years, recommendations regarding the use of phage preparations for treatment and prevention of bacterial infections have been based on the analysis of scientific-clinical researches, clearly certifying the high efficacy of phage therapy and prophylaxis without any contraindications and complications.

Numerous clinical trials corroborated that application of phage preparations, in case of its administration at the onset of the disease and susceptibility of the pathogenic bacteria to the bacteriophage, improves a patient's condition after 24 hours in 64%, and 48 hours in 93-95%.

It has to be mentioned that substitution of antibiotics with phage preparations for treatment of various bacterial infections, cause developing of antibiotic-susceptible cells in bacterial (natural and clinical) populations. There are some clinical cases described, where antibiotic resistant bacterial strains were replaced with antibiotic sensitive ones after treating patients with phage preparations. ( 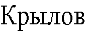 B.H., 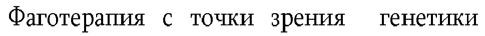 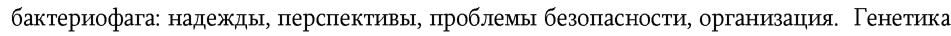 , 2001, 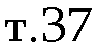 , Nº7, c.869-887).

It is worth noting, that application of therapeutic bacteriophage preparations during bacterial infections is recommended as follows:
- As monotherapy - at the early, initial stage of the disease;
- In combination with other antibacterial medicines (including antibiotics) at the acute phase of the disease;
- In conjunction with pathogenetic-oriented methods of treatment, while ongoing the second course of etiological therapy after inefficacy of the first one (antibiotics and chemotherapy);
- As monotherapy or in conjunction with immunoprotectors - in case of re-excretion of bacteria (bacterial carriage);
- During the treatment of gut dysbiosis, when *Staphylococcus aureus,* hemolytic *Escherichia coli* or other conditionally pathogenic bacteria are intensively multiplied in the intestinal microflora (Intestiphage, Pyophage);
- For prevention of bacterial intestinal infections (dysentery, salmonellosis, typhoid and paratyphoid fever (*S.paratyphi* A,B);
- In case of inefficiency of antibiotics (antibiotic resistance) and chronic recurrent infections;
- In case of any contraindications for antibiotics (antibiotic-associated diarrhea, injury of liver, kidney and other organs);
- In case of allergy to chemical antibacterial medications;
- May also be used in pregnant and breastfeeding women, newborns and infants (as contraindications, side effects, general toxic, immunosuppressive, embryotoxic and teratogenic effects of bacteriophages have not been detected).

High therapeutic potential of phage preparations, their multi-species pattern and polyvalent composition, strict specificity and complete safety - assigns a high priority to consumption of therapeutic bacteriophage preparations in practical medicine as one of the alternatives to antibiotics for treatment and prevention of bacterial infections. ( 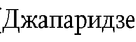 , 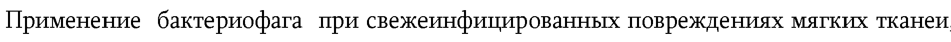 , 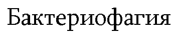 , 1955, 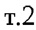 . c.407-409; 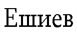 A.M., 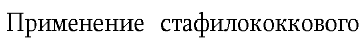 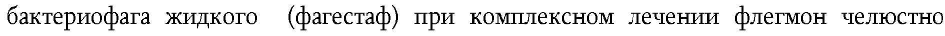 - 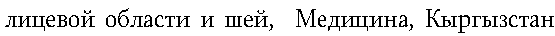 , 2009, Nº6, c.23-25; 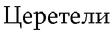 E.B. 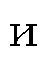 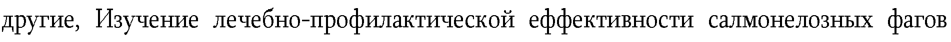 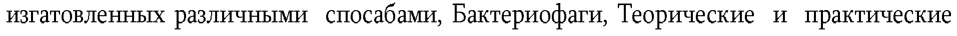 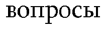 , 1985; 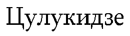 A. Π., 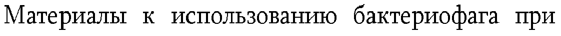 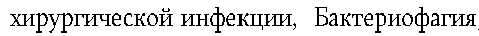 , 1957, 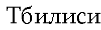 , c.363-372; Appelmans R., Le bacteriophage dans l'organisme, Comp. Rend. Soc. de Biol., 1921, Paris, 85, p.722-724; Brüssow H., Phage therapy: the Escherichia coli experience, 10.1099/mic.0.27849-0 Microbiology, 2005, vol.151, N7, p.2133-2140; Carl R., Merril C., Dean Scholl and Sankar L. et al., The prospect for bacteriophage therapy in Western medicine. Proc. Nat. Acad. Sci., 2003, USA, v.2; Carlton R.M., Phage therapy: Past history and future prospects. J. Arch. Immunol. Ther. Exper., 1999, 47, p.267-274; Gorski A., Dabrowska K., Switala-Jelen K. et al., New insights into the possible role of bacteriophages in host defence and disease. Med. Immunol., 2003, 2, 2; Gorski A., Hirszfeld L., Phage therapy - adventages over antibiotics? The Lancet, 2000, 356, 1418; Gorski A., Kniotek M., Perkowska-Ptasinska A et al., Bacteriophages and transplantation tolerance, Transplant. and Proc., 2006, 38 (1): 331-333; Gorski A., Weber-Dabrowska B., The potential role of endogenous bacteriophage in controlling invading pathogens., Cell. Mol. Life, 2005, Sci.62, 511; Kutter E, Abedon ST., et al., Phage therapy in clinical practice: treatment of human infections, Curr Pharm Biotechnol., 2010 Jan; 11(1):69-86; Miedzybrodski R., Fortuna W., Weber-Dabrowska B., Gorski A., Bacterial viruses against viruses pathogenic for man. Virus Res., 2005, 110, 1; Rhoads DD, Wolcott RD, Kuskowski MA, Wolcott BM, Ward LS, Sulakvelidze A, Bacteriophage therapy of venous leg ulcers in humans: results of a phase I safety trial., J. Wound Care., 2009 Jun, 18(6):237-8, 240-3; Sulakvelidze A., Alavidze Z., Vorris JG., Bacteriophage therapy (minireview), Antimicrob Agents Chemother., 2001, 45(3): 649-659; Weber-Dabrowska B., Mulczyk M., Gorski A., Bacteriophage therapy of bacterial infections: an update of our Institute experience., J. Arch. Immun. Exptl., 2000, 48, 547; Yan J., Fan X., Xie J., Emerging biomedicines based on bacteriophages, Crit Rev Eukaryot Gene Expr., 2013, 23(4): 299-308; Zhang J., Hong Y. et al., Physiological and Molecular Characterization of Salmonella Bacteriophages Previously Used in Phage Therapy. J. Food Prot., 2015, 78(12):2143-9, doi: 10.4315/0362-028X.JFP-14-350).

Therapeutic and prophylactic bacteriophage preparations may be monocomponent (monovalent or polyvalent) or polycomponent (combined polyvalent). The monocomponent preparations comprise one or more true virulent bacteriophages, homologous with a single species of bacteria (serotype, serovar). The examples of the monocomponent preparations are: *Staphylococcus* phage (Phagestaph), *Pseudomonas aeruginosa* phage (Phagepy), *Salmonella* phage (Phagesal, polyvalent), dysentery phage (Phagedys), *Klebsiella, Vibrio cholerae*, *Serratia* bacteriophages, etc. (Burbutashvili T., Golijashvili A., Dzuliashvili M., Chkhartishvili S., Bondirev I., Saralidze D., Japarashvili N., Investigation of some biological properties of Enterococcus strains identified in Tbilisi in 2003-2004, Proceedings of Georgian Academy of Sciences, Biological series A, 2005, volume 31, N1, pp.13 -21; Chanishvili Z., Chanishvili T., Cholokashvili N., Dzuliashvili M., Gachechiladze K. Proteus Phage-O29 and P113: A Comparative Study of Host Range, Antigenic Determinations and Structural Variations, August 22 2001, The Evergreen St. College, 14th International Phage biology Meeting; Dzuliashvili M., Gabitashvili K. et al., Study of therapeutic potential of the experimental Pseudomonas Bacteriophage Preparation, Georgian Medical News, 2007, 6(147), ISSN-1512-0112, Tbilisi, Georgia; Dzuliashvili M., Golijashvili A., et al. Isolation, taxonomy and comparative characterization of bacteriophages active to conditionally - pathogenic Pseudomonas aeruginosa strains, Proceedings of Georgian Academy of Sciences, Biological series, 2004, volume 25, N6, pp. 885-893; Dzuliashvili M., Golijashvili A., et al., Allocation, systematics and comparative charachteristics of bacteriophages, active to the conditionally-pathogenic microorganisms of Pseudomonas aeruginosa, Proceedings of Georgian Academy of Sciences, Biological series A, 2004, volume 30, 6, pp.885-893; Dzuliashvili M., Golijashvili A. et al., Comparative characteristics of potentially-therapeutic bacteriophages, active to opportunistic pathogens of P.aeruginosa by virulence test, International seminar - Perspective of usage of bacteriophage preparations for prevention and treatment of infections caused by pathogenic and conditioned pathogenic microorganizms, Materials, 2005.10-11.11, pp.32, 82; Dzuliashvili M., Kutter E., Gabitashvili K., Gachechiladze K., Construction and Characterization Therapeutic Bacteriophages with Attack Pseudomonas aeruginosa Strains Isolated from USA Patients with Cystic Fibrosis15th Evergreen International Phage Biology Meeting, 2003, poster 31, Olympia, WA, USA; Dzuliashvili M., et al., Construction and Characterization Therapeutic Bacteriophages with Attack Pseudomonas aeruginosa Strains Isolated from USA Patients with Cystic Fibrosis, 15th Evergreen International Phage Biology Meeting, 2003 Aug., Olympia, WA, USA, Poster 31; Dzuliashvili M. et al., Selection and Development of therapeutic phage cocktails for treatment of P.aeruginosa infections, International Seminar - Perspective of usage of bacteriophage preparations for prevention and treatment of infections caused by pathogenic and conditioned pathogenic microorganizms, 2005.10-11.11, Poster 10, Abstract book, p.103; Gabitashvili K., Marina Dzuliashvili, Tamila Meskhi, Tina Kvelashvili, Ketevan Gachechiladze, Elizabeth Kutter, Selection and Construction of Experimental Races of Specific Bacteriophages Active Against Pseudomonas aeruginosa Isolated in USA from Various Infection Pathologies, 16th Evergreen International Phage Biology Meeting, Aug 7-12th 2005, poster T-51, Olympia, WA, USA; Golijashvili A., Dzuliashvili M., Gachechiladze K., Isolation and characterization of therapeutic phages specific for Serattia marcescens, Proceedings of Georgian Academy of Sciences, Biological series, 1999, volume 25, 1, pp.14-26; Golijashvili A., Dzuliashvili M., Gachechiladze K., Bondirev I., Study of Serratia phages with some of virulence tests, Proceedings of Georgian Academy of Sciences, Biological series A, 2005, volume 31, 2, pp.261-268; Golijashvili A., Dzuliashvili M., Gachechiladze K., et al., Some aspects of selection of treatment - prophylactic Serratia marcescens bacteriophages, Proceedings of Georgian Academy of Sciences, 2004, Biological series A, volume 30, N6, pp.787-797; Golijashvili A., Nikogosova N., Gachechiladze K., Production of new treatment - prophylactic bacteriophage preparations active to Serratia marcescens and Enterobacter aerogenes, Topical questions in Microbiology and Virology, Tbilisi, Collection of works, ' 1996, volume IX, pp.68-71; Golijashvili A., Dzuliashvili M., et al., Production and identification of potentially therapeutic bacteriophage of Serratia marcescens, International seminar - Perspectives for the use of bacteriophage preparations for the prevention and treatment of infections caused by pathogenic and opportunistic pathogenic microorganisms, Materials, 2005, p.41, 9210-11, November, 2005, Tbilisi; Golijashvili A., et al., 15th Evergreen International Phage Biology Meeting., 2003 Aug. 2-10, Olympia, WA, USA, Poster 10, Interaction of serologic specificity and therapeutic potential of bacteriophages; Golijashvili A. et al., 2nd International ASM-FEMS Conference on Enterococci, Bacteriophages as alternative antibacterial remedy against enterococcal infections, 2005, American Society for Microbiology, B 116, p. 95; Golijashvili A. et al., International Seminar, Perspectives of usage of bacteriophages preparations for prevention and treatment of infections caused by pathogenic and conditional pathogenic microorganisms, ISTC, 10-11 XI, 2005, Tbilisi, Georgia - Comparative characteristics of the potentially therapeutic Bacteriophages active against opportunistic microorganisms P. aeruginosa by virulence tests, pp.82-83; Golijashvili A. et al., International Seminar, Perspectives of usage of bacteriophages preparations for prevention and treatment of infections caused by pathogenic and conditional pathogenic microorganisms, ISTC, 10-11 XI, 2005, Tbilisi, Georgia - Applying perspective of bacteriophages for diagnostics, treatment and prevention of the infections induced by the genera Klebsiella and Enterobacter, p.90; Golijashvili A et al., International Seminar, Perspectives of usage of bacteriophages preparations for prevention and treatment of infections caused by pathogenic and conditional pathogenic microorganisms, ISTC, 10-11 XI, 2005, Tbilisi, Georgia - Development of Serratia marcescens Bacteriophages and definition of their therapeutic potential, p.92; Golijashvili A. et al., 5-th International Conference, Bioresearches and viruses, 2007, Kiev: Diarrheal agents and antibacterial preparations; Bacteriophages as a remedy for treatment of diarrhea, Problems of diarrhea).

Polycomponent preparations comprise multiple true virulent bacteriophages, homologous with various species (two or more) of bacteria (serotype, serovariant).

According to Prior Art, there are known a number of polycomponent preparations, some of which are described in the following sources: Orynbassarova K. K., Shakim G. A., et al., Application Pyobacteriophage in complex treatment of children with pneumonia, Clinical observation, Vestnik of NSU, 2012, 10(4): 122-125; Orynbassarova K. K., Shakim G. A., Comparative studies of clinical effectiveness of different antibacterial remedies for treatment of children with pneumonia, International congress, Health for everyone: Prophylaxis, Treatment, Rehabilitation, 2012, Almaty, pp 272-273.

Even though there are a lot of phage containing preparations nowadays, development of a highly efficient medication for the infectious diseases, caused by various microbes, is still extremely important. The fact that a plurality of diseases, conditioned by multidrug-resistant microbial associations, has been noticeably increased - makes the above-mentioned issue more significant. Therefore, treatment of such diseases requires polycomponent preparations, comprising true virulent bacteriophages, which have the ability to jointly, thoroughly cover the whole specter of disease-causing microbe.

### Summary of the Invention

One particular embodiment of the invention relates to an antimicrobial composition against gastrointestinal infections, which comprises:
a) Bacteriophage strains, specific to *Shigella flexneri:* DSM 32619 and DSM 32620, which are deposited in German Collection of Microorganisms and Cell Cultures (DSMZ),
b) Bacteriophage strains, specific to *Shigella sonnei:* DSM 32621 and DSM 32622, which are deposited in German Collection of Microorganisms and Cell Cultures (DSMZ),
c) Bacteriophage strain, specific to *Salmonella choleraesuis* - DSM 32625, which is deposited in German Collection of Microorganisms and Cell Cultures (DSMZ),
d) Bacteriophage strain, specific to *Salmonella newport* - DSM 32624, which is deposited in German Collection of Microorganisms and Cell Cultures (DSMZ),
e) Bacteriophage strain, specific to *Salmonella paratyhi* A - DSM 32623, which is deposited in German Collection of Microorganisms and Cell Cultures (DSMZ),
f) Bacteriophage strain, specific to *Salmonella typhimurium* - DSM 32626, which is deposited in German Collection of Microorganisms and Cell Cultures (DSMZ),
g) Bacteriophage strain, specific to *Salmonella paratyhi* B - DSM 32627, which is deposited in German Collection of Microorganisms and Cell Cultures (DSMZ),
h) Bacteriophage strain, specific to *Salmonella heidelberg* - DSM 32628, which is deposited in German Collection of Microorganisms and Cell Cultures (DSMZ),
i) Bacteriophage strains, specific to *Escherichia coli:* DSM 32612, DSM 32611 and DSM 32610, which are deposited in German Collection of Microorganisms and Cell Cultures (DSMZ),
j) Bacteriophage strains, specific to *Proteus vulgaris:* DSM 32613, DSM 32614 and DSM 32615, which are deposited in German Collection of Microorganisms and Cell Cultures (DSMZ),
k) Bacteriophage strains, specific to *Staphylococcus aureus* DSM 32631, DSM 32629 and DSM 32630, which are deposited in German Collection of Microorganisms and Cell Cultures (DSMZ),
l) Bacteriophage strains, specific to *Pseudomonas aeruginosa'.* DSM 32616, DSM 32618 and DSM 32617, which are deposited in German Collection of Microorganisms and Cell Cultures (DSMZ),
m) Bacteriophage strains, specific to *Enterococcus.* DSM 32632 and DSM 32633, which are deposited in German Collection of Microorganisms and Cell Cultures (DSMZ), and optionally a pharmaceutically acceptable excipient.

In another embodiment of the invention, the above-mentioned composition may have a form, which will be selected from the following group: liquid formulation, spray, tablet, powder, capsule, cream and suppository.

In another embodiment of the invention, the above-mentioned composition is used for treatment and/or prevention of intestinal infections.

In another embodiment of the invention, the above-mentioned composition is used for treatment and/or prevention of intestinal infections in humans.

In another embodiment of the invention, the above-mentioned composition is used for treatment and/or prevention ofintestinal infections in animals and poultry.

In another embodiment of the invention, the above-mentioned composition is used for treatment and/or prevention of dysentery (shigellosis), salmonellosis, escherichiosis *(E.coli-*infection), dyspepsia, dysbiosis, foodbome toxicoinfections, enteritis, gastroenteritis, colitis, enterocolitis and gastroenterocolitis in humans.

Another embodiment of the invention is defined as isolated bacteriophage strains, constituents of the above-mentioned composition, which have a lytic activity against *Shigella Pexneri* and are selected from the following group: DSM 32619 and DSM 32620. All the above-named strains are deposited in German Collection of Microorganisms and Cell Cultures (DSMZ).

Another embodiment of the invention is defined as isolated bacteriophage strains, constituents of the above-mentioned composition, which have a lytic activity against *Shigella sonnei* and are selected from the following group: DSM 32621 and DSM 32622. All the above-named strains are deposited in German Collection of Microorganisms and Cell Cultures (DSMZ).

Another embodiment of the invention is defined as an isolated bacteriophage strain DSM 32625, which is constituent of the above-mentioned composition, has a lytic activity against *Salmonella choleraesuis* and is deposited in German Collection of Microorganisms and Cell Cultures (DSMZ).

Another embodiment of the invention is defined as an isolated bacteriophage strain DSM 32624, which is constituent of the above-mentioned composition, has a lytic activity against *Salmonella newport* and is deposited in German Collection of Microorganisms and Cell Cultures (DSMZ).

Another embodiment of the invention is defined as an isolated bacteriophage strain DSM 32623, which is constituent of the above-mentioned composition, has a lytic activity against *Salmonella paratyphi* A and is deposited in German Collection of Microorganisms and Cell Cultures (DSMZ).

Another embodiment of the invention is defined as an isolated bacteriophage strain DSM 32626, which is constituent of the above-mentioned composition, has a lytic activity against *Salmonella typhimurium* and is deposited in German Collection of Microorganisms and Cell Cultures (DSMZ).

Another embodiment of the invention is defined as an isolated bacteriophage strain DSM 32627, which is constituent of the above-mentioned composition, has a lytic activity against *Salmonella paratyphi* B and is deposited in German Collection of Microorganisms and Cell Cultures (DSMZ).

Another embodiment of the invention is defined as an isolated bacteriophage strain DSM 32628, which is constituent of the above-mentioned composition, has a lytic activity against *Salmonella heidelberg* and is deposited in German Collection of Microorganisms and Cell Cultures (DSMZ).

Another embodiment of the invention is defined as isolated bacteriophage strains, constituents of the above-mentioned composition, which have a lytic activity against *Enterococcus* and are selected from the following group: DSM 32632 and DSM 32633. All the above-named strains are deposited in German Collection of Microorganisms and Cell Cultures (DSMZ).

### Description of the Figures

Fig.1 depicts an electron microscopic image of DSM 32619;
Fig.2 depicts an electron microscopic image of DSM 32620;
Fig.3 depicts an electron microscopic image of DSM 32621;
Fig.4 depicts an electron microscopic image of DSM 32622;
Fig.5 depicts an electron microscopic image of DSM 32625;
Fig.6 depicts an electron microscopic image of DSM 32624;
Fig.7 depicts an electron microscopic image of DSM 32623;
Fig.8 depicts an electron microscopic image of DSM 32626;
Fig.9 depicts an electron microscopic image of DSM 32627;
Fig.10 depicts an electron microscopic image of DSM 32628;
Fig.11 depicts an electron microscopic image of DSM 32612;
Fig.12 depicts an electron microscopic image of DSM 32611;
Fig.13 depicts an electron microscopic image of DSM 32610;
Fig.14 depicts an electron microscopic image of DSM 32613;
Fig.15 depicts an electron microscopic image of DSM 32614;
Fig.16 depicts an electron microscopic image of DSM 32615;
Fig.17 depicts an electron microscopic image of DSM 32631;
Fig.18 depicts an electron microscopic image of DSM 32629;
Fig.19 depicts an electron microscopic image of DSM 32630;
Fig.20 depicts an electron microscopic image of DSM 32616;
Fig.21 depicts an electron microscopic image of DSM 32618;
Fig.22 depicts an electron microscopic image of DSM 32617;
Fig.23 depicts an electron microscopic image of DSM 32632;
Fig.24 depicts an electron microscopic image of DSM 32633;
Fig.25 depicts restriction fragment length polymorphism (RFLP) profiles of DSM 32619, DSM 32620, DSM 32621 and DSM 32622, using the enzyme *Afl* II.
Fig.26 depicts restriction fragment length polymorphism (RFLP) profiles of DSM 32624, DSM 32625, DSM 32627 and DSM 32623, using the enzyme *Hind III.*
Fig.27 depicts restriction fragment length polymorphism (RFLP) profiles of DSM 32626 and DSM 32628, using the enzyme *Spe* I*.*
Fig.28 depicts restriction fragment length polymorphism (RFLP) profile of DSM 32627 using the enzyme *Spe* I.
Fig.29 depicts RFLP profiles of DSM 32612, DSM 32610 and DSM 32611, using the enzymes: *EcoR* V and Aflll.
Fig.30 depicts RFLP profiles of DSM 32613, DSM 32614 and DSM 32615, using the enzyme *Hind III.*
Fig.31 depicts RFLP profiles of DSM 32613, DSM 32614 and DSM 32615, using the enzyme *Afl* II.
Fig.32 depicts restriction fragment length polymorphism (RFLP) profiles of DSM 32631, DSM 32629 and DSM 32630, using the enzymes: *Eco*R I, *EcoR* V, *Hind* III and *Spe* I.
Fig.33 depicts RFLP profile of DSM 32616 using the enzyme *Hind III.*
Fig.34 depicts RFLP profiles of DSM 32618 and DSM 32617, using the enzymes: *EcoR* V and *Hind* III.
Fig.35 depicts RFLP profiles of DSM 32632 and DSM 32633, using the enzyme *Hind* III.

### Detailed Description of the Invention

### Antimicrobial composition and preparations thereof

According to one of the preferred embodiments of the invention, there is provided an antimicrobial composition, which contains:
a) Bacteriophage strains, specific to *Shigella flexneri:* DSM 32619 and DSM 32620, which are deposited in German Collection of Microorganisms and Cell Cultures (DSMZ),
b) Bacteriophage strains, specific to *Shigella sonnei:* DSM 32621 and DSM 32622, which are deposited in German Collection of Microorganisms and Cell Cultures (DSMZ),
c) Bacteriophage strain DSM 32625, specific to *Salmonella choleraesuis,* which is deposited in German Collection of Microorganisms and Cell Cultures (DSMZ),
d) Bacteriophage strain DSM 32624, specific to *Salmonella newport,* which is deposited in German Collection of Microorganisms and Cell Cultures (DSMZ),
e) Bacteriophage strain DSM 32623, specific to *Salmonella paratyphi* A, which is deposited in German Collection of Microorganisms and Cell Cultures (DSMZ),
f) Bacteriophage strain DSM 32626, specific to *Salmonella typhimurium*, which is deposited in German Collection of Microorganisms and Cell Cultures (DSMZ),
g) Bacteriophage strain DSM 32627, specific to *Salmonella paratyphi* B, which is deposited in German Collection of Microorganisms and Cell Cultures (DSMZ),
h) Bacteriophage strain DSM 32628, specific to *Salmonella heidelberg*, which is deposited in German Collection of Microorganisms and Cell Cultures (DSMZ),
i) Bacteriophage strains, specific to *Escherichia coli:* DSM 32612, DSM 32611 and DSM 32610, which are deposited in German Collection of Microorganisms and Cell Cultures (DSMZ),
j) Bacteriophage strains, specific to *Proteus vulgaris*: DSM 32613, DSM 32614 and DSM 32615, which are deposited in German Collection of Microorganisms and Cell Cultures (DSMZ),
k) Bacteriophage strains, specific to *Staphylococcus aureus*: DSM 32631, DSM 32629 and DSM 32630, which are deposited in German Collection of Microorganisms and Cell Cultures (DSMZ),
l) Bacteriophage strains, specific to *Pseudomonas aeruginosa:* DSM 32616, DSM 32618 and DSM 32617, which are deposited in German Collection of Microorganisms and Cell Cultures (DSMZ),
m) Bacteriophage strains, specific to *Enterococcus*: DSM 32632 and DSM 32633, which are deposited in German Collection of Microorganisms and Cell Cultures (DSMZ) and optionally a pharmaceutically acceptable excipient.

According to the invention, a phage, which is a component of the composition, refers to the deposited bacteriophage and progeny derived therefrom, the genetic profile of which is substantially equivalent to the profile of said bacteriophage.

Based on the data of World Health Organization, 90% of the gastrointestinal infections are caused by pathogenic and conditionally pathogenic bacteria: *Salmonella, Shigella, E.coli, Proteus, Pseudomonas aeruginisa, Staphylococcus, Enterococcus.*

*Shigella flexneri* and *Shigella sonnei* cause shigellosis. Shigellosis accounts for a large number of acute intestinal infections. Worldwide, *Shigella* causes 165 million cases of disease annually, of which 600.000 are fatal. Children are affected in 75-80% of all shigellosis cases. *Shigella* infection is the most common in children between the ages of 1-6. Complications of severe dysentery in younger children may include nephritis, toxic and hypovolemic shock, rectal prolapse, bleeding, invagination or peritonitis. Bronchopneumonia, stomatitis, pyoderma, furuncolosis, osteomyelitis, urinary tract infections, hemolytic-uremic syndrome with acute kidney failure, non-purulent arthritis and Reyter's syndrome may also occur; *Shigella*-induced bacteremia causes death in 50% of patients.

The main diseases, caused by *Salmonelleae,* may be divided into three groups: typhoid and paratyphoid fever, gastroenteritis and septicemia.

Typhoid and paratyphoid fever - (typhus abdominalis, *S.paratyphi* A and *S.paratyphi* B infections) - belong to the group of acute infections, caused by *Salmonella typhi, Salmonella Paratyphi* A, *Salmonella schottmulleri.* These diseases are characterized by fever, general intoxication, bacteremia, hepatosplenomegaly, enteritis and specific injury of intestinal lymphatic system. *Salmonelleae* release endotoxin when they are disrupted, which induces symptoms of general intoxication and plays an important role in development of small intestinal ulcers, leukopenia and potentially toxic shock syndrome (TSS).

Salmonellosis is an acute infectious disease, which is caused by *Salmonelleae* and characterized by a wide range of clinical manifestations, including asymptomatic carriage or even septic forms. It mainly affects gastrointestinal tract (gastroenteritis, colitis). The leading causes of salmonellosis in children are: *S. typhimurium, S. enteritidis, S. infantis, S. anatum, S. heidelberg* and others. Septic form of salmonellosis is characterized by especially severe course of the disease. The illness does not respond to antibiotic-therapy well. Secondary purulent foci are frequently formed in the musculoskeletal system (osteomyelitis, arthritis, spondylitis). Sometimes salmonellosis-related infective endocarditis, aortitis with development of late aortic aneurysm, purulent meningitis; rarely - liver abscess, purulent strumitis and infection of the ovarian cyst may also occur. Severe forms of the disease may cause dehydration, as well as toxic shock syndrome (TSS). Salmonella is estimated to cause up to 1.000.000 food poisoning cases, 19.000 hospitalizations and 380 deaths in the United States every year.

*Esherichia coli* causes enteritis, urethritis, cystitis, pyelonephritis, cholecystitis, peritonitis, septicemia, meningitis in children, wound infections etc. *E.coli* is a main causative microorganism of acute intestinal infections. Escherichiosis (synonym - intestinal *E.coli* infection) elicits the symptoms of gastroenteritis and gastroenterocolitis.

Diarrheagenic *E.coli* is divided into 5 categories: enterotoxigenic (ETEC), enteroinvasive (EIEC), enteropathogenic (EPEC), enterohemorrhagic (EHEC) and enteroadherent (EAEC). Enterotoxigenic *E.coli* is a main causative agent of traveler's diarrhea. It also is a common cause of gastroenteritis in young children. Enterohemorrhagic *E.coli* causes hemorrhagic colitis. Except for diarrhea, enterohemorrhagic *E.coli* causes developing of hemolytic-uremic syndrome (HUS). Enterohemorrhagic strains of *E.coli* is distinguished by high virulence and pathogenicity.

The most common etiologic agent of urogenital disorders during pregnancy (68.8-80%) is *E. coli.*

*Proteus vulgaris* causes urinary tract infections (UTIs), abscesses, meningitis, gastroenteritis, burn wound infections, as well as secondary septic lesions after surgical procedures and burn injuries.

*Staphylococcus aureus* is a major pathogen responsible for neonatal nosocomial infections (61.5%). It is a main causative agent of pneumonia (24%), sepsis (24.6-28%), meningitis, skin infections (39%) and bacterial sinusitis (24-80%). Sputum culture of the patients, having cystic fibrosis (mucoviscidosis), which has been a global issue over the past 20 years, most commonly reveals *S.aureus* (63-65%) in young age groups. *Staphylococcus aureus* is detected in 12,1-12,5% of urogenital disorders during pregnancy, such as: pyelonephritis, cystitis, urethritis, bacteriuria, colpitis, endocervicitis, endometritis, salpingo-oophoritis.

Staphylococcal enterocolitis may be the primary manifestation of infection or develop secondary to sepsis or other underlying conditions (pneumonia, meningitis, omphalitis etc.). The disease most frequently occurs in weak, premature babies up to 6 months of age, with different accompanying diseases.

*Pseudomonas aeruginosa* causes 15-20% of nosocomial infections, 16-20% of hospital-acquired pneumonia (HAP) and 20-25% of purulent-surgical infections. One-third of urogential tract infections (UTIs) is also resulted from *P.aeruginosa. P.aeruginosa* is a causative agent of keratitis, endocarditis, enteritis, conjunctivitis, otitis, meningitis, bacteremia/septicemia, perirectal and rectal abscesses, osteomielitis and arthritis. Mortality rate for bacteremia-septicemia is 35-75%. The most common pathogens detected with a sputum culture of the patients, having cystic fibrosis (mucoviscidosis) are mucoid (virulent), multidrug-resistant *P.aeruginosa* strains. 90% of chronic infections conditioned by the above-mentioned microorganism is fatal.

*Enterococci* (*Enterococcus faecium* and *Enterococcus faecalis*) cause enteritis, colitis, urinary tract infections (UTIs), endocarditis, bacteremia-septicemia, neonatal sepsis. Most of the enterococcal infections are considered as nosocomial. 80% of the strains, isolated from hospitals, is vancomycin-resistant *Enterococcus faecium.*

The aforementioned data of World Health Organization (WHO) and Georgian National Center for Disease Control and Public Health (NCDC), as well as the fact that an infection is rarely caused by a single microbial species, led to the development of an effective antimicrobial composition. True virulent bacteriophages, constituents of the composition, result in lysis of the main aforementioned pathogens and are highly efficient in treating and preventing the infections, caused by microbial associations. It is worth mentioning, that different types of bacteriophages, constituents of the composition, do not reveal antagonistic action to each other, which proves their strict specificity once more.

In a preferred embodiment of the invention, titer of the phages and phage concentrates in the composition are as follows:

| Phage type | Phage titer | Titer of phage concentrate |
|---|---|---|
| DSM 32619 | 2×10¹⁰ | 1×10¹¹ |
| DSM 32620 | 2×10¹⁰ | 2×10¹¹ |
| DSM 32621 | 3×10¹⁰ | 4×10¹¹ |
| DSM 32622 | 5×10⁹ | 1×10¹¹ |
| DSM 32625 | 3×10¹⁰ | 2×10¹¹ |
| DSM 32624 | 1×10¹⁰ | 3×10¹¹ |
| DSM 32623 | 1×10⁹ | 4×10¹⁰ |
| DSM 32626 | 3×10⁸ | 1×10¹⁰ |
| DSM 32627 | 4×10⁹ | 3×10¹¹ |
| DSM 32628 | 2×10¹⁰ | 4×10¹¹ |
| DSM 32612 | 1×10¹⁰ | 3×10¹¹ |
| DSM 32611 | 2×10⁹ | 1×10¹¹ |
| DSM 32613 | 1×10⁹ | 4×10¹⁰ |
| DSM 32614 | 6×10⁸ | 1×10¹¹ |
| DSM 32615 | 2×10⁹ | 1×10¹¹ |
| DSM 32610 | 4×10¹⁰ | 4×10¹¹ |
| DSM 32631 | 3×10¹⁰ | 3×10¹¹ |
| DSM 32629 | 1×10¹⁰ | 4×10¹¹ |
| DSM 32630 | 5×10¹⁰ | 2×10¹¹ |
| DSM 32616 | 4×10¹⁰ | 5×10¹¹ |
| DSM 32618 | 1×10¹⁰ | 1×10¹¹ |
| DSM 32617 | 2×10⁹ | 2×10¹¹ |
| DSM 32632 | 1×10⁹ | 1×10¹¹ |
| DSM 32633 | 3×10⁸ | 5×10¹⁰ |

According to the invention, the composition contains bacteriophages and optionally a carrier. The composition may have a liquid or lyophilized powder formulation. The preparation, produced on the basis of the composition, may have a form of injection, infusion, spray, tablet, capsule, cream or suppository. The preparation should preferably comprise a pharmaceutically acceptable carrier.

In order to produce the needed form (e.g. solution for injection, nasal spray etc.) or before using it, the composition of lyophilized powder formulation may be suspended in water for injection, buffered solution, 5% glucose solution, glycerine, dextran, polyethylene glycol, sorbitol or any kind of solution, that maintain phage viability and are not toxic to humans.

According to the invention, sachets, tablets and capsules may be produced on the basis of the powder composition using the well-known pharmaceutical technologies. The above-mentioned preparations may contain stabilizers, conservatives, additional active ingredients, e.g. antibiotics. Tablets and capsules may be produced by the technology, which will ensure the release of the active ingredient in the intestines.

The preparation, made on the basis of the composition, may have a liquid form for oral administration, e.g. suspensions, solutions, emulsions or syrups. Such formulations may contain suspending agents, emulsifiers, conservatives, aromatizing agents, sweeteners etc.

On the basis of the composition topical preparations may also be developed. Such preparations include creams and suppositories. They contain bases, which are well-known in the pharmaceutical industry, maintain phage viability and are not toxic to humans.

### Bacteriophages, components of the composition

The composition comprises isolated bacteriophages. Cultivation of the isolated bacteriophages is carried out separately from the environment. Thus, each strain of the isolated bacteriophage is pure and practically does not include other bacteriophage mixtures.

Deposited bacteriophages, constituent of the composition, are specific to the targeted bacteria and have the ability to lyse them.

According to the invention, the concept of a bacteriophage, constituent of the composition, includes the deposited bacteriophages, as well as progeny thereof, genetic profile of which are essentially equivalent to the profile of said bacteriophages, thus, they fully maintain specificity to the targeted bacteria. The above-mentioned phage progeny may have some genetic variations, the limits of which corresponds with the standards for "closely related microorganisms", developed by Tenover (Tenover, et al. (1995) "Interpreting Chromosomal DNA Restriction Patterns Produced by Pulsed-Field Gel Electrophoresis Criteria for Bacterial Strain Typing." J. Clin. Microbiol.33: 2233-2239). Bacteriophages, needed to produce the composition, are cultivated by the method, technique and materials of which are well-known for the specialists of the above-mentioned field. More specifically, each production strain of the targeted bacteria, susceptible to the deposited bacteriophages, is cultivated in a culture medium and then inoculated with its specific bacteriophages (deposited bacteriophages which are specific to the aforementioned bacteria) at the pre-determined optimal multiplicity of infection (MOI). After incubation and bacterial lysis, bacteriophages are collected, purified and concentrated, which result in obtaining the phages, necessary for the composition production. The steps of purification and concentration include various systems of filtration and centrifugation, which are well-known for the field specialists (Adams, M. H. (1959). Methods of study bacterial viruses. Bacteriophages. London, Interscience Publishers, Ltd.: 443-519).

Determination of a particular bacteriophage concentration is carried out by phage titration assay. If higher concentration of the bacteriophages - specific to the particular bacterial strain - is needed, concentrating is carried out by filtration and centrifugation, and if less concentration is required, it is suspended in water or buffer. Finally, in order to produce the composition, progeny of each deposited bacteriophage strain, obtained by this method, is combined into a single phage cocktail.

The composition contains the following bacteriophage strains, specific to *Shigella flexneri*: DSM 32619 and DSM 32620, which are deposited in German Collection of Microorganisms and Cell Cultures (DSMZ).

The optimal conditions for propagation of the above-mentioned bacteriophages are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of these bacteriophages were determined. Morphology of the bacteriophages - DSM 32619 and DSM 32620 were investigated with the aid of electron microscope. The interaction between the above-mentioned phages and their host cells, particularly, adsorbtion rate of bacteriophages onto the host cells in short period of time (5min), as well as adsorbtion rate constant - K were also studied.

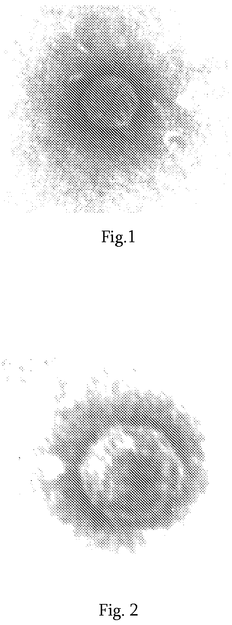

The data of the above-noted assays are shown in table 1 (the table shows bacterial strains of the collection, maintained by JSC "Biochimpharm"), while figures 1-2 depict electron-microscopic images of the above-mentioned bacteriophages.

Genomes of the bacteriophages - DSM 32619 and DSM 32620, particularly, restriction fragment length polymorphism profiles (RFLP) were also studied. In order to determine RFLP profiles, restriction enzyme - *Afl* II was used.

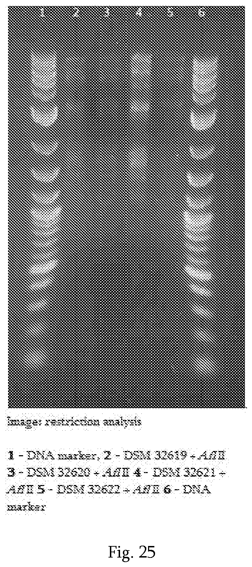

RFLP profiles of the above-mentioned bacteriophages are shown in figure 25.

The composition contains the following bacteriophage strains, specific to *Shigella sonnei:* DSM 32621 and DSM 32622, which are deposited in German Collection of Microorganisms and Cell Cultures (DSMZ).

The optimal conditions for propagation of the above-mentioned bacteriophages are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of these bacteriophages were determined. Morphology of the bacteriophages - DSM 32621 and DSM 32622 were investigated with the aid of electron microscope. The interaction between the above-mentioned phages and their host cells, particularly, adsorbtion rate of the bacteriophages onto the host cells in short period of time (5min), as well as adsorbtion rate constant - K were also studied.

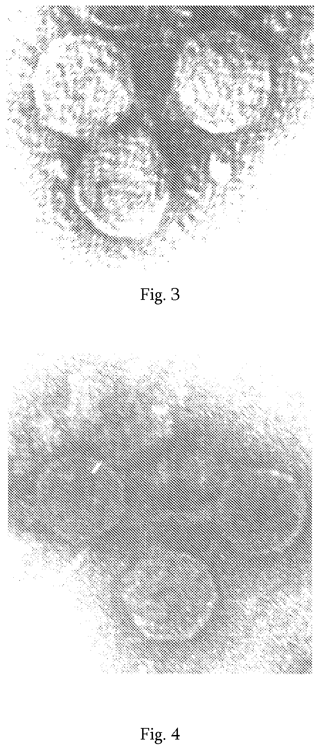

The data of the above-noted assays are shown in table 2 (the table shows bacterial strains of the collection, maintained by JSC "Biochimpharm"), while figures 3-4 depict electron-microscopic images of the above-mentioned bacteriophages.

Genomes of the bacteriophages - DSM 32621 and DSM 32622, particularly, restriction fragment length polymorphism profiles (RFLP) were also studied. In order to determine RFLP profiles, restriction enzyme - *Afl* II was used.

RFLP profiles of the above-mentioned bacteriophages are shown in figure 25.

The composition contains the following bacteriophage strain, specific to *Salmonella choleraesuis:* DSM 32625 - which is deposited in German Collection of Microorganisms and Cell Cultures (DSMZ).

The optimal conditions for propagation of the above-mentioned bacteriophage are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of this bacteriophage were determined. Morphology of the bacteriophage - DSM 32625 was investigated with the aid of electron microscope. The interaction between the above-mentioned phage and its host cell, particularly, adsorbtion rate of the bacteriophage onto the host cell in short period of time (5min), as well as adsorbtion rate constant - K were also studied.

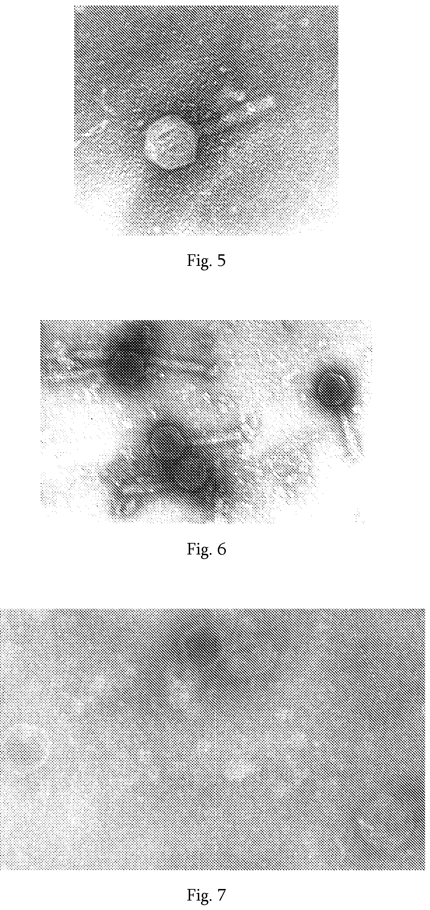

The data of the above-noted assays are shown in table 3 (the table shows bacterial strains of the collection, maintained by JSC "Biochimpharm"), while figure 5 depicts electron-microscopic image of the above-mentioned bacteriophage.

Genome of the bacteriophage - DSM 32625, particularly, restriction fragment length polymorphism profile (RFLP) was also studied. In order to determine RFLP profile, restriction enzyme - *Hind* III was used.

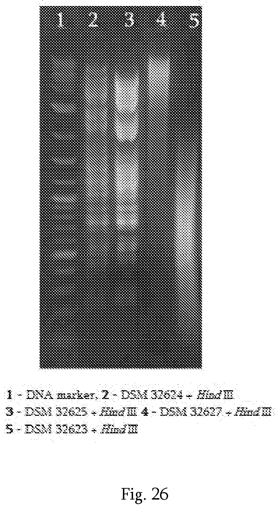

RFLP profiles of the above-mentioned bacteriophages are shown in figure 26.

The composition contains the following bacteriophage strain, specific to *Salmonella newport* DSM 32624 - which is deposited in German Collection of Microorganisms and Cell Cultures (DSMZ).

The optimal conditions for propagation of the above-mentioned bacteriophage are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of this bacteriophage were determined. Morphology of the bacteriophage - DSM 32624 was investigated with the aid of electron microscope. The interaction between the above-mentioned phage and its host cell, particularly, adsorbtion rate of the bacteriophage onto the host cell in short period of time (5min), as well as adsorbtion rate constant - K were also studied.

The data of the above-noted assays are shown in table 3 (the table shows bacterial strains of the collection, maintained by JSC "Biochimpharm"), while figure 6 depicts electron-microscopic image of the above-mentioned bacteriophage.

Genome of the bacteriophage - DSM 32624, particularly, restriction fragment length polymorphism profile (RFLP) was also studied. In order to determine RFLP profile, restriction enzyme - *Hind III* was used.

RFLP profiles of the above-mentioned bacteriophages are shown in figure 26.

The composition contains the following bacteriophage strain, specific to *Salmonella paratyphi* A: DSM 32623 - which is deposited in German Collection of Microorganisms and Cell Cultures (DSMZ).

The optimal conditions for propagation of the above-mentioned bacteriophage are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of this bacteriophage were determined. Morphology of the bacteriophage - DSM 32623 was investigated with the aid of electron microscope. The interaction between the above-mentioned phage and its host cell, particularly, adsorbtion rate of the bacteriophage onto the host cell in short period of time (5min), as well as adsorbtion rate constant - K were also studied.

The data of the above-noted assays are shown in table 4 (the table shows bacterial strains of the collection, maintained by JSC "Biochimpharm"), while figure 7 depicts electron-microscopic image of the above-mentioned bacteriophage.

Genome of the bacteriophage - DSM 32623, particularly, restriction fragment length polymorphism profile (RFLP) was also studied. In order to determine RFLP profile, restriction enzyme - *Hind III* was used.

RFLP profiles of the above-mentioned bacteriophages are shown in figure 26.

The composition contains the following bacteriophage strain, specific to *Salmonella typhimurium:* DSM 32626 - which is deposited in German Collection of Microorganisms and Cell Cultures (DSMZ).

The optimal conditions for propagation of the above-mentioned bacteriophage are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of this bacteriophage were determined. Morphology of the bacteriophage - DSM 32626 was investigated with the aid of electron microscope. The interaction between the above-mentioned phage and its host cell, particularly, adsorbtion rate of the bacteriophage onto the host cell in short period of time (5min), as well as adsorbtion rate constant - K were also studied.

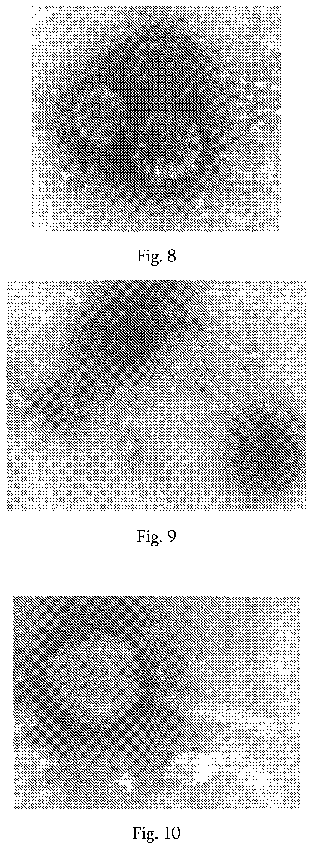

The data of the above-noted assays are shown in table 4 (the table shows bacterial strains of the collection, maintained by JSC "Biochimpharm"), while figure 8 depicts electron-microscopic image of the above-mentioned bacteriophage.

Genome of the bacteriophage - DSM 32626, particularly, restriction fragment length polymorphism profile (RFLP) was also studied. In order to determine RFLP profile, restriction enzyme - *Spe* I was used.

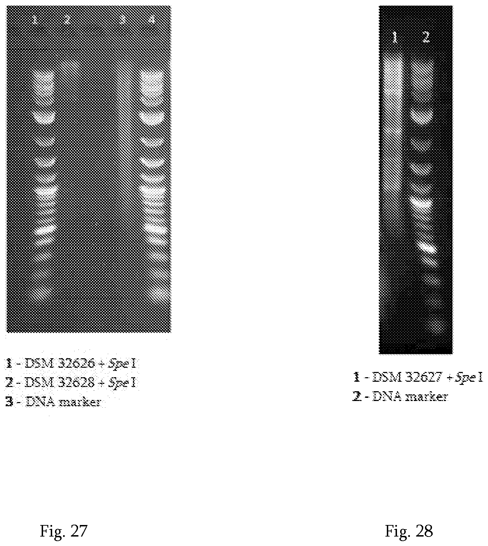

RFLP profiles of the above-mentioned bacteriophages are shown in figure 27.

The composition contains the following bacteriophage strain, specific to *Salmonella paratyphi* B: DSM 32627 - which is deposited in German Collection of Microorganisms and Cell Cultures (DSMZ).

The optimal conditions for propagation of the above-mentioned bacteriophage are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of this bacteriophage were determined. Morphology of the bacteriophage - DSM 32627 was investigated with the aid of electron microscope. The interaction between the above-mentioned phage and its host cell, particularly, adsorbtion rate of the bacteriophage onto the host cell in short period of time (5min), as well as adsorbtion rate constant - K were also studied.

The data of the above-noted assays are shown in table 5 (the table shows bacterial strains of the collection, maintained by JSC "Biochimpharm"), while figure 9 depicts electron-microscopic image of the above-mentioned bacteriophage.

Genome of the bacteriophage - DSM 32627, particularly, restriction fragment length polymorphism profile (RFLP) was also studied. In order to determine RFLP profile, restriction enzyme - *Spe* I was used.

RFLP profiles of the above-mentioned bacteriophages are shown in figure 28.

The composition contains the following bacteriophage strain, specific to *Salmonella heidelberg.* DSM 32628 - which is deposited in German Collection of Microorganisms and Cell Cultures (DSMZ).

The optimal conditions for propagation of the above-mentioned bacteriophage are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of this bacteriophage were determined. Morphology of the bacteriophage - DSM 32628 was investigated with the aid of electron microscope. The interaction between the above-mentioned phage and its host cell, particularly, adsorbtion rate of the bacteriophage onto the host cell in short period of time (5min), as well as adsorbtion rate constant - K were also studied.

The data of the above-noted assays are shown in table 5 (the table shows bacterial strains of the collection, maintained by JSC "Biochimpharm"), while figure 10 depicts electron-microscopic image of the above-mentioned bacteriophage.

Genome of the bacteriophage - DSM 32628, particularly, restriction fragment length polymorphism profile (RFLP) was also studied. In order to determine RFLP profile, restriction enzyme - *Spe* I was used.

RFLP profiles of the above-mentioned bacteriophages are shown in figure 27.

The composition contains the following bacteriophage strains, specific to *Escherichia coli:* DSM 32612, DSM 32611 and DSM 32610 - which are deposited in German Collection of Microorganisms and Cell Cultures (DSMZ).

The optimal conditions for propagation of the above-mentioned bacteriophages are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of these bacteriophages were determined. Morphology of the bacteriophages - DSM 32612, DSM 32611 and DSM 32610 - was investigated with the aid of electron microscope. The interaction between the above-mentioned phages and their host cells, particularly, adsorbtion rate of the bacteriophages onto the host cells in short period of time (5min), as well as adsorbtion rate constant - K were also studied.

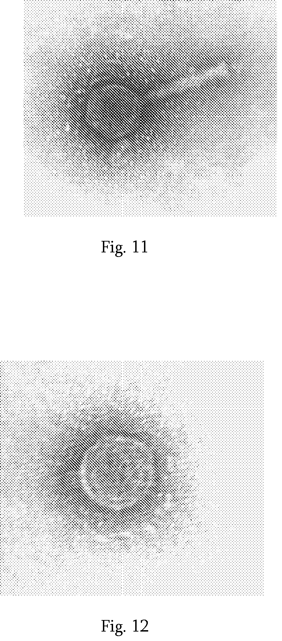

The data of the above-noted assays are shown in table 6 (the table shows bacterial strains of the collection, maintained by JSC "Biochimpharm"), while figures 11-13 depict electron-microscopic images of the above-mentioned bacteriophages.

Genomes of the bacteriophages - DSM 32612, DSM 32611 and DSM 32610, particularly, restriction fragment length polymorphism profiles (RFLP) were also studied. In order to determine RFLP profiles, restriction enzymes: *EcoR* V and *Afl* II were used.

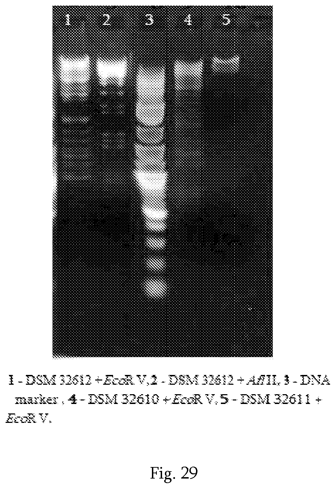

RFLP profiles of the above-mentioned bacteriophages are shown in figure 29.

The composition contains the following bacteriophage strains, specific to *Proteus vulgaris:* DSM 32613, DSM 32614 and DSM 32615 - which are deposited in German Collection of Microorganisms and Cell Cultures (DSMZ).

The optimal conditions for propagation of the above-mentioned bacteriophages are: pH - 7-7,4; temperature - 30-37°C; culture media: pancreatic hydrolizate of fish meal or soya broth; multiplicity of infection (MOI) - 0,2; cultivation duration - 15-18h.

Plaque morphology and lytic activity of these bacteriophages were determined. Morphology of the bacteriophages - DSM 32613, DSM 32614 and DSM 32615 - was investigated with the aid of electron microscope. The interaction between the above-mentioned phages and their host cells, particularly, adsorbtion rate of the bacteriophages onto the host cells in short period of time (5min), as well as adsorbtion rate constant - K were also studied.

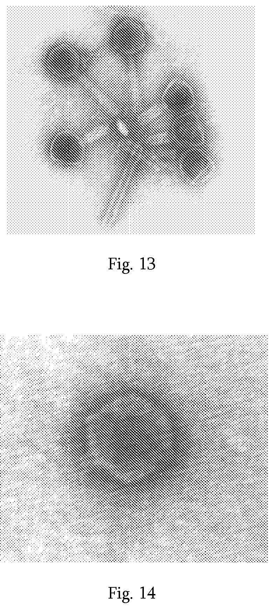

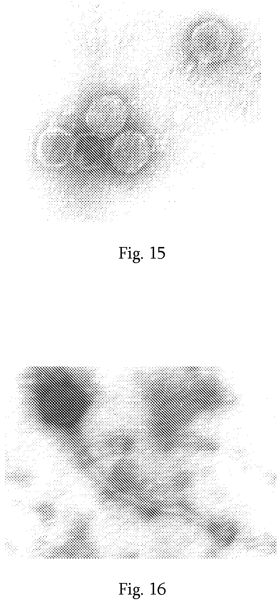

The data of the above-noted assays are shown in table 7 (the table shows bacterial strains of the collection, maintained by JSC "Biochimpharm"), while figures 14-16 depict electron-microscopic images of the above-mentioned bacteriophages.

Genomes of the bacteriophages - DSM 32613, DSM 32614 and DSM 32615, particularly, restriction fragment length polymorphism profiles (RFLP) were also studied. In order to determine RFLP profiles, restriction enzymes: *Hind* III and *Afl* II were used.

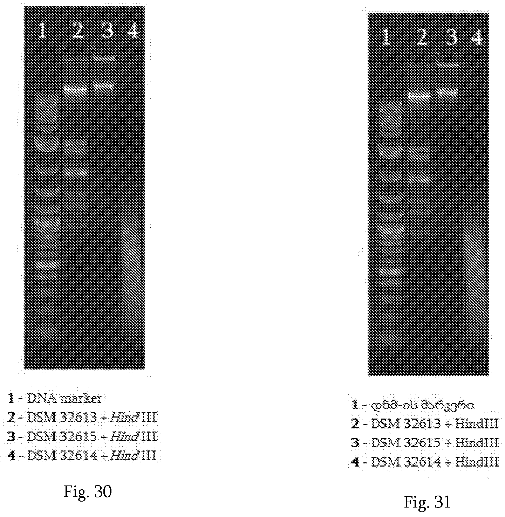

RFLP profiles of the above-mentioned bacteriophages are shown in figures 30 and 31.

The composition contains the following bacteriophage strains, specific to *Staphylococcus aureus* DSM 32631, DSM 32629 and DSM 32630 - which are deposited in German Collection of Microorganisms and Cell Cultures (DSMZ).

The optimal conditions for propagation of the above-mentioned bacteriophages are: pH - 7-7,4; temperature - 30-37°C; culture media: pancreatic hydrolizate of fish meal or soya broth; multiplicity of infection (MOI) - 0,8; cultivation duration - 15-18h.

Plaque morphology and lytic activity of these bacteriophages were determined. Morphology of the bacteriophages - DSM 32631, DSM 32629 and DSM 32630 - was investigated with the aid of electron microscope. The interaction between the above-mentioned phages and their host cells, particularly, adsorbtion rate of the bacteriophages onto the host cells in short period of time (5min), as well as adsorbtion rate constant - K were also studied.

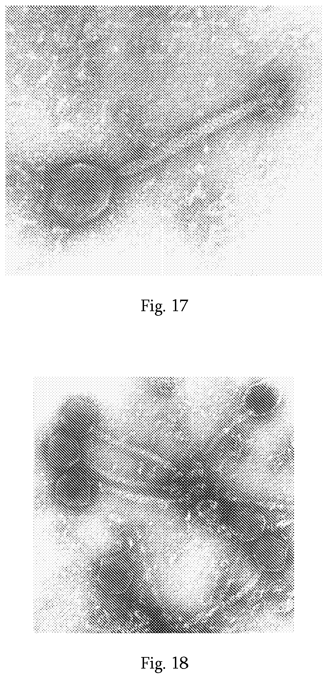

The data of the above-noted assays are shown in table 8 (the table shows bacterial strains of the collection, maintained by JSC "Biochimpharm"), while figures 17-19 depict electron-microscopic images of the above-mentioned bacteriophages.

Genomes of the bacteriophages - DSM 32631, DSM 32629 and DSM 32630, particularly, restriction fragment length polymorphism profiles (RFLP) were also studied. In order to determine RFLP profiles, restriction enzymes: *EcoR* I, EcoR V, Hind III, Spe I, *Afl* II were used.

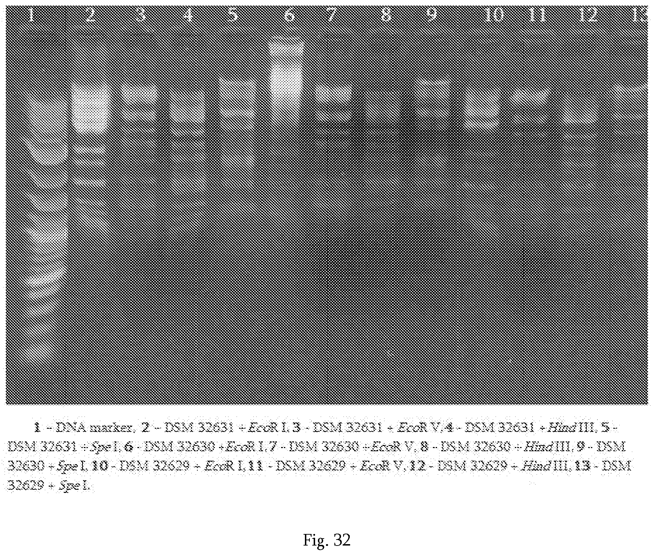

RFLP profiles of the above-mentioned bacteriophages are shown in figure 32.

The composition contains the following bacteriophage strains, specific to *Pseudomonas aeruginosa:* DSM 32616, DSM 32618 and DSM 32617- which are deposited in German Collection of Microorganisms and Cell Cultures (DSMZ).

The optimal conditions for propagation of the above-mentioned bacteriophages are: pH - 7-7,4; temperature - 30-37°C; culture media: pancreatic hydrolizate of fish meal or soya broth; multiplicity of infection (MOI) - 2,0; cultivation duration - 15-18h.

Plaque morphology and lytic activity of these bacteriophages were determined. Morphology of the bacteriophages - DSM 32616, DSM 32618 and DSM 32617- was investigated with the aid of electron microscope. The interaction between the above-mentioned phages and their host cells, particularly, adsorbtion rate of the bacteriophages onto the host cells in short period of time (5min), as well as adsorbtion rate constant - K were also studied.

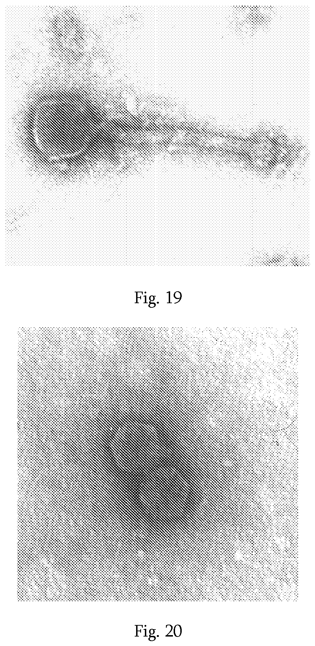

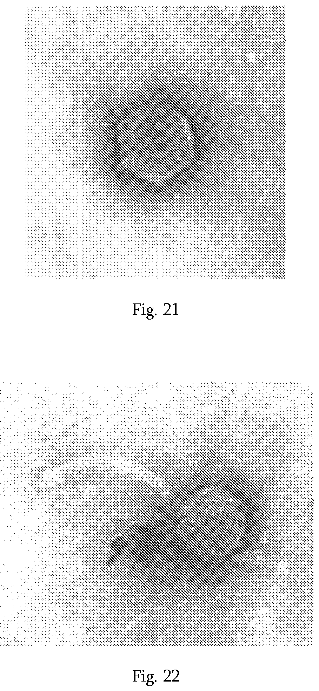

The data of the above-noted assays are shown in table 9 (the table shows bacterial strains of the collection, maintained by JSC "Biochimpharm"), while figures 20-22 depict electron-microscopic images of the above-mentioned bacteriophages.

Genomes of the bacteriophages - DSM 32616, DSM 32618 and DSM 32617, particularly, restriction fragment length polymorphism profiles (RFLP) were also studied. In order to determine RFLP profiles, restriction enzymes: *EcoR* V and *Hind* III were used.

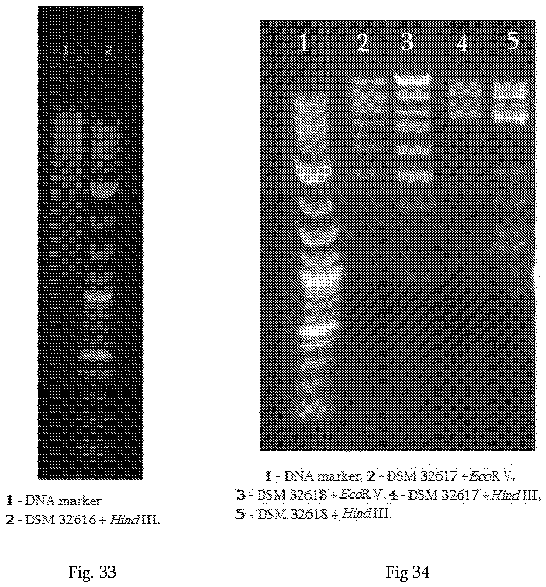

RFLP profiles of the above-mentioned bacteriophages are shown in figures 33 and 34.

The composition contains the following bacteriophage strains, specific to *Enterococcus:* DSM 32632 and DSM 32633 - which are deposited in German Collection of Microorganisms and Cell Cultures (DSMZ).

The optimal conditions for propagation of the above-mentioned bacteriophages are: pH - 7-7,4; temperature - 30-37°C; culture media: pancreatic hydrolizate of fish meal or soya broth; multiplicity of infection (MOI) - 0,3; cultivation duration - 15-18h.

Plaque morphology and lytic activity of these bacteriophages were determined. Morphology of the bacteriophages - DSM 32632 and DSM 32633 - was investigated with the aid of electron microscope. The interaction between the above-mentioned phages and their host cells, particularly, adsorbtion rate of the bacteriophages onto the host cells in short period of time (5min), as well as adsorbtion rate constant - K were also studied.

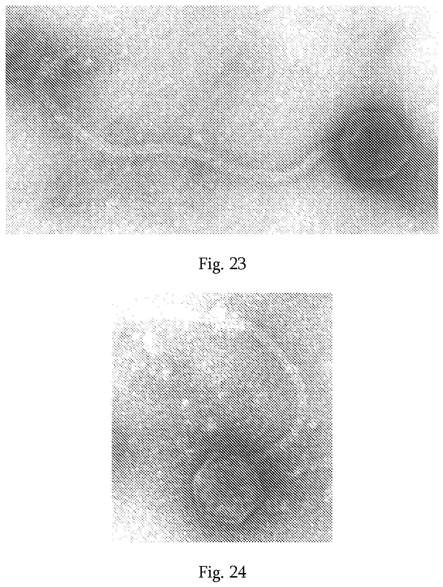

The data of the above-noted assays are shown in table 10 (the table shows bacterial strains of the collection, maintained by JSC "Biochimpharm"), while figures 23-24 depict electron-microscopic images of the above-mentioned bacteriophages.

Genomes of the bacteriophages - DSM 32632 and DSM 32633, particularly, restriction fragment length polymorphism profiles (RFLP) were also studied. In order to determine RFLP profiles, restriction enzyme *Hind* III were used.

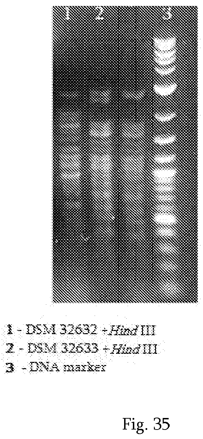

RFLP profiles of the above-mentioned bacteriophages are shown in figure 35.

*In vitro* lytic activity of bacteriophages, which were specific to 24 bacterial strains, constituents of the composition, were also studied. Particularly, lytic activity of bacteriophages, specific to 306 bacterial strains of the collection, maintained by JSC "Biochimpharm", as well as 159 bacterial strains of international collections and various countries (Spain, Germany, Australia, etc.) were investigated.

The results of phage lytic activity against bacterial strains of the collection, maintained by JSC "Biochimpharm" are shown in table 11. According to the table, *in vitro* efficiency - spectrum of lytic activity of bacteriophages, which are constituents of the above-mentioned composition, against their homologous bacterial strains are as follows: *Shigella* (65 strains) - 78-89%; *Salmonella* (57 strains) - 74-88%; *E.coli* (36 strains) - 80,6-94,4%; *Proteus* (7 strains) - 100%; *S.aureus* (36 strains) - 89,9-94,4%; *P.aeruginosa* (38 strains) - 76,3-94,7%; *Enterococcus* (67 strains) - 91-92,5%. Screening analysis showed that spectrum of the lytic activity of these 24 bacteriophages, constituents of the composition, overlaps each other; thus, *in vitro* efficiency - spectrum of the lytic activity of the batceriophages, constituents of the composition, against 306 bacterial strains of the collection, maintained by JSC "Biochimpharm", is 100%.

Lytic activity of bacteriophages, constituents of the composition, against bacterial strains of international collections and various countries (Spain, Germany, Australia, USA) is as follows: against *Staphylococcus aureus* strains - 95,2%; against *E.coli* strains - 75 %; against *Proteus* strains -100%; against *P. aeruginosa* strains - 75%; against *Enterococcus* strains - 95%; against *Salmonella* strains - 76%; against *Shigella* strains - 100%.

### The use of the composition for treatment and prevention

The composition is used for treatment and prevention.

The composition is indicated to be used for treatment of the following diseases: dysentery (shigellosis), salmonellosis, escherichiosis (*E.coli* infection), dyspepsia, dysbiosis, foodbome toxicoinfections, enteritis, gastroenteritis, colitis, enterocolitis, gastroenterocolitis.

The composition is recommended to be used for prevention during outbreaks of intestinal infections in food establishments, places of mass gatherings, closed groups (kindergartens, schools, hospitals, etc.), military units (under field conditions and during military operations), and during natural disasters.

Except the above-mentioned conditions, the composition may also be used in phage diagnostics, phage indication and phage prophylaxis. Its application can be directed towards normalizing the ecological condition, environmental sanitation, agricultural and aquaculture products.

For reduction, elimination and prevention of colonization with pathogenic bacteria, food products may be processed by the composition, proposed by the invention. The preparations, produced on the basis of the composition, may be used in manufacturing facilities, retirement homes, kindergartens, etc. for the purpose of environmental sanitation and prevention of bacterial colonization.

The composition may be used as an additive in cosmetic products (creams, lotions, gels, etc.).

Besides, *in vivo* therapeutic efficiency of the composition was also studied. Over the course of phage therapy (5-7 days) with the composition in children with intestinal infections of mild and medium severity, complete elimination of pathogenic and conditionally pathogenic microbes: hemolytic *E.coli* strains (in 100% of the patients) and *S.aureus* (in 87%), as well as partial eradication of *Proteus vulgaris, Klebsiella, Serratia* - were achieved. Yet it is noteworthy that in 50% of patients, who did not undergo anti-fungal therapy, revealed elimination of *Candida* spp. infections.

The clinical trials studying the composition (50 patients with the diagnosis of acute bacterial intestinal infection of medium severity, etiologic agents: *S.enteritidis, S.typhimurium, S.flexner*) revealed significant improvement in 48 hours after treatment initiation in 95% of patients (reduction in the intensity and frequency of intoxication, abdominal pain, lack of appetite, malaise, diarrhea). After a short-course monotherapy with the composition (3-5 days), full recovery of patients, proven by clinical and laboratory findings, was achieved.

Moreover, efficiency of the composition was compared with the effectiveness of antibiotics, Sextaphage (RU2410084 (FEDERAL NOE GUP NPOB MED IMMUNOBIOLOGICHESKIM PREPARATAM MIKROGEN MIN ZDRAVOOKHRANENIJA RF) 27.01.2011) and Pyophage (RU2036232 (UFIM Nil VAKTSIN I SYVOROTOK) 27.05.1995). Efficiency of bacteriophages, constituents of the composition, during intestinal infections, which were caused by antibiotic-resistant bacterial strains, is 75-100%, particularly: 95,2% against *Staphylococcus aureus* strains, 75% against *E.coli* strains, 100% against *Proteus* strains, 75% against *P.aeruginosa* strains, 95% against *Enterococcus* strains, 76% against *Salmonella* strains, 100% against *Shigella* strains. Meanwhile, effectiveness of Sextaphage and Pyophage is 43-93%, particularly, 70-93% against *Staphyloccccus aureus* strains, 68-75% against *E.coli* strains, 55-76% against *Proteus* strains, 43-61,5% against *P.aeruginosa* strains. As for antibiotic therapy, its efficacy does not exceed 64% (Sulakvelidze A., Alavidze Z., Vorris J.G., Bacteriophage therapy (minireview), Antimicrob Agents Chemother., 2001, 45(3): 649-659).

Hence, the composition, proposed by the invention, is highly efficient medium to treat and prevent diseases, caused by various microbes, especially, microbial associations.

**Table 1**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32619 | *Shigella flexneri*-1 | Podoviridae; C1 | 6,5-7,6 mm big colonies with a light center and a halo | | |
| | | | capside - 60 nm | | 2×10¹⁰ | K₅=5,4×10⁻⁹ |
| | | | tail - 14 nm (x 250 000) | | 1×10¹¹ | 93,3% |
| 2 | DSM 32620 | *Shigella flexneri-*1268 | Podoviridae;C1 | 8.5-9 mm big colonies with a small, light center and a big halo | | K₅=5×10⁻⁹ |
| | | | capside - 68 nm | | 2×10¹⁰ | |
| | | | tail - 4 nm (x 250 000) | | 2×10¹¹ | 92,1% |

**Table 2**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32621 | *Shigella sonnei*-24 | Podoviridae; C1 | 8,5 mm big colonies with a light center and; big halo | | |
| | | | capside - 58 nm | | 3×10¹⁰ | K₅=5,98×10⁻⁹ |
| | | | tail - 8 nm (x 250 000) | | 4×10¹¹ | 95% |
| 2 | DSM 32622 | *Shigella sonnei*-48 | Podoviridae; C 1 | 6,5 mm big colonies with a small, light cente and a rounded halo | | |
| | | | capside - 54.35 nm | | 5×10⁹ | K₅=7×10⁻⁹ |
| | | | tail - 26 nm (x 230 000) | | 1×10¹¹ | 97% |

**Table 3**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32625 | *Salmonella choleraesuis -* 747 | Myoviridae; A1 | 1-1,5mm small light colonies | | |
| | | | capside - 80 nm | | 3×10⁹ | K₅=7.6×10⁻⁹ |
| | | | tail - 112nm (x 250 000) | | 2×10¹¹ | 85.2% |
| 2 | DSM 32624 | *Salmonella newport-*285 | Myoviridae; A1 | 1,5-2mm small light colonies | | K₅=9.2×10⁻⁹ |
| | | | capside - 68 nm | | 1×10¹⁰ | 90% |
| | | | tail - 120 nm (x 250 000) | | 3×10¹¹ | |

**Table 4**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32623 | *Salmonella* P.A. - 222 | Podoviridae; C1 | 7-7,5mm big colonies with a light center and a big halo | | |
| | | | capside - 55.3 nm | | 1×10⁹ | K₅=8,14×10⁻⁹ |
| | | | tail - 25.5 nm (x 235 000) | | 4×10¹⁰ | 86.9% |
| 2 | DSM 32626 | *S.typhimurium* 14028 | Podoviridae; C1 | 5mm big colonies with a light center and a big halo | 3×10⁸ | K₅=8.84×10⁻⁹ |
| | | | capside - 60 nm | | | 89% |
| | | | tail - 8 nm (x 250 000) | | 1×10¹⁰ | |

**Table 5**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32627 | *Salmonella* P.B-24 | Siphoviridae; B1 | 2,5-3mm medium sized, light colonies | | |
| | | | capside - 80 nm | | 4×10⁹ | K₅=7.774×10⁻⁹ |
| | | | tail - 200 nm (x 250 000) | | 3×10¹¹ | 85.7% |
| 2 | DSM 32628 | *Salmonella heidelberg-*67 | Myoviridae; A1 | 0,5mm dot sized, ligh colonies | 2×10¹⁰ | K₅=6.62×10⁻⁹ |
| | | | capside - 96 nm | | | 81% |
| | | | tail - 128 nm (x 250 000) | | 4×10¹¹ | |

**Table 6**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32612 | *E.coli -* O_{18ac}B₂₁H₇ | Myoviridae; A1 | 3mm medium sized, transparent colonies | | |
| | | | capside - 64 nm | | 1×10¹⁰ | K₅=4,43×10⁻⁹ |
| | | | tail - 112 nm (x 250 000) | | 3×10¹¹ | 88.57% |
| 2 | DSM 32611 | *E.coli -* O₅₅B₅ | Podoviridae; C1 | 6mm big colonies with a light center and a halo | 2×10⁹ | K₅=3,76×10⁻⁹ |
| | | | capside - 56 nm | | 1×10¹¹ | 84.8% |
| | | | tail - 16 nm (x 250 000) | | | |
| 3 | DSM 32610 | *E.coli -* O₂₆B₆ | Myoviridae; A1 | 2-2,5mm medium-sized, transparent colonies | | |
| | | | capside - 72 nm | | 4×10¹⁰ | K₅-3,58×10⁻⁹ |
| | | | tail - 120 nm (x 250 000) | | 4×10¹¹ | 83.3% |

**Table 7**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32613 | *Proteus vulgarls*-1 | Podoviridae; C1 | 4mm colonies with 2mm light center and a halo | | |
| | | | Size of the hexagonal head- 56.5nm | | 1×10⁹ | K₅=1,23×10⁻⁹ |
| | | | Legth of the short tail-8.7nm (x 230 000) | | 4×10¹⁰ | 84.12% |
| 2 | DSM 32614 | *Proteus vulgaris-*125 | Podoviridae; C1 | 4.5-5mm big colonies with a light center and a halo | | |
| | | | Size of the elongated head - 61 nm | | 6×10⁸ | K₅=6,49×10⁻⁹ |
| | | | Legth of the short tail - 13 nm (x 230 000) | | 1×10¹¹ | 80.3% |
| 3 | DSM 32615 | *Proteus vulgaris*-509 | Siphoviridae; B1 | 6-6.5mm big colonies with small, light center and an irregularly edged halo | | |
| | | | Size of the head - 82.6 nm | | 2×10⁹ | K₅=9,31×10⁻⁹ |
| | | | Legth of the long, flexible tail - 391.3 nm (x 230 000) | | 1×10¹¹ | 90.3% |

**Table 8**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32631 | *Staphylococcus aureus* 53 | Myoviridae; A1 | 1,5 mm small, light colonies | | |
| | | | Capside - 98 nm | | 3×10¹⁰ | K₅=3,8×10⁻⁹ |
| | | | Tail - 257 nm (x 245 000) | | 3×10¹¹ | 85% |
| 2 | DSM 32629 | *Staphylococcus aureus* 14 | Siphoviridae; B1 | 2 mm small, light colonies | | |
| | | | Capside - 80 nm | | 1×10¹⁰ | K₅=3,38×10⁻⁹ |
| | | | Tail 216 nm (x 250 000) | | 4×10¹¹ | 81.6% |
| 3 | DSM 32630 | *Staphylococcus aureus* 51 | Myoviridae; A1 | 1 mm small, light colonies | | |
| | | | Icosahedric capside - 87 nm | | 5×10¹⁰ | K₅=3,7×10⁻⁹ |
| | | | Complex tail - 256.5 nm (x 230 000) | | 2×10¹¹ | 84% |

**Table 9**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32616 | *P.aeruginosa* - 157 | Podoviridae; C1 | 7mm big, light colonies with a light center and an irregularly edged halo | | |
| | | | Capside - 56 nm | | 4×10¹⁰ | K₅=4×10⁻⁹ |
| | | | Tail - 16 nm (x 250 000) | | 5×10¹¹ | 86% |
| 2 | DSM 32618 | *P.aeruginosa -* 27853 | Podoviridae; C1 | 4mm big colonies with a transparent center and a small halo | | |
| | | | Capside - 68 nm | | 1×10¹⁰ | K₅=3,89×10⁻⁹ |
| | | | Tail - 8 nm (x 250 000) | | 1×10¹¹ | 85.7% |
| 3 | DSM 32617 | *P.aeruginosa* - 573 | Siphoviridae; B1 | 3-3,5mm medium sized colonies with a light center and a halo | | |
| | | | Capside - 64nm | | 2×10⁹ | K₅=3,4×10⁻⁹ |
| | | | Tail - 140 nm (x 250 000) | | 2×10¹¹ | 82% |

**Table 10**

| N | Phage Name | Name of the Host Cell | Virion Morphology | Plaque Morphology | Phage Titer; Concentrate Titer (PFU/ml) | Adsorption K (ml/min) & % in 5 min |
|---|---|---|---|---|---|---|
| 1 | DSM 32632 | *Enterococcus -* 50 | Siphoviridae; B1 | 1 mm small, light colonies | | |
| | | | Capside - 87 nm | | 1×10⁹ | K₅=1,72×10⁻⁸ |
| | | | Flexible, long tail - 448 nm | | 1×10¹¹ | 83% |
| | | | (x 230 000) | | | |
| 2 | DSM 32633 | *Enterococcus -* 317 | Siphoviridae; B1 | 2 mm small, light colonies | | K₅=1,94×10⁻⁸ |
| | | | Capside - 74 nm | | 3×10⁸ | |
| | | | Flexible, long tail (curved) - 226 nm | | 5×10¹⁰ | 85.7% |
| | | | (x 230 000) | | | |

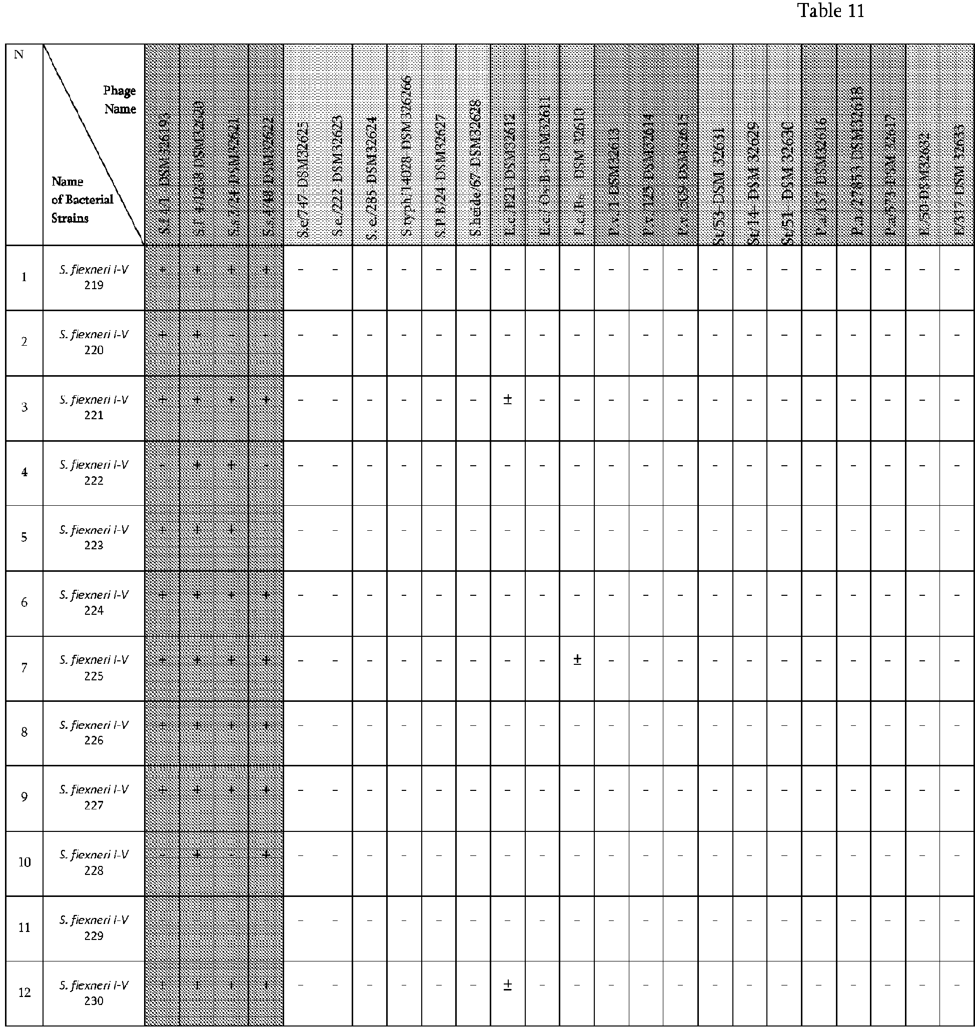

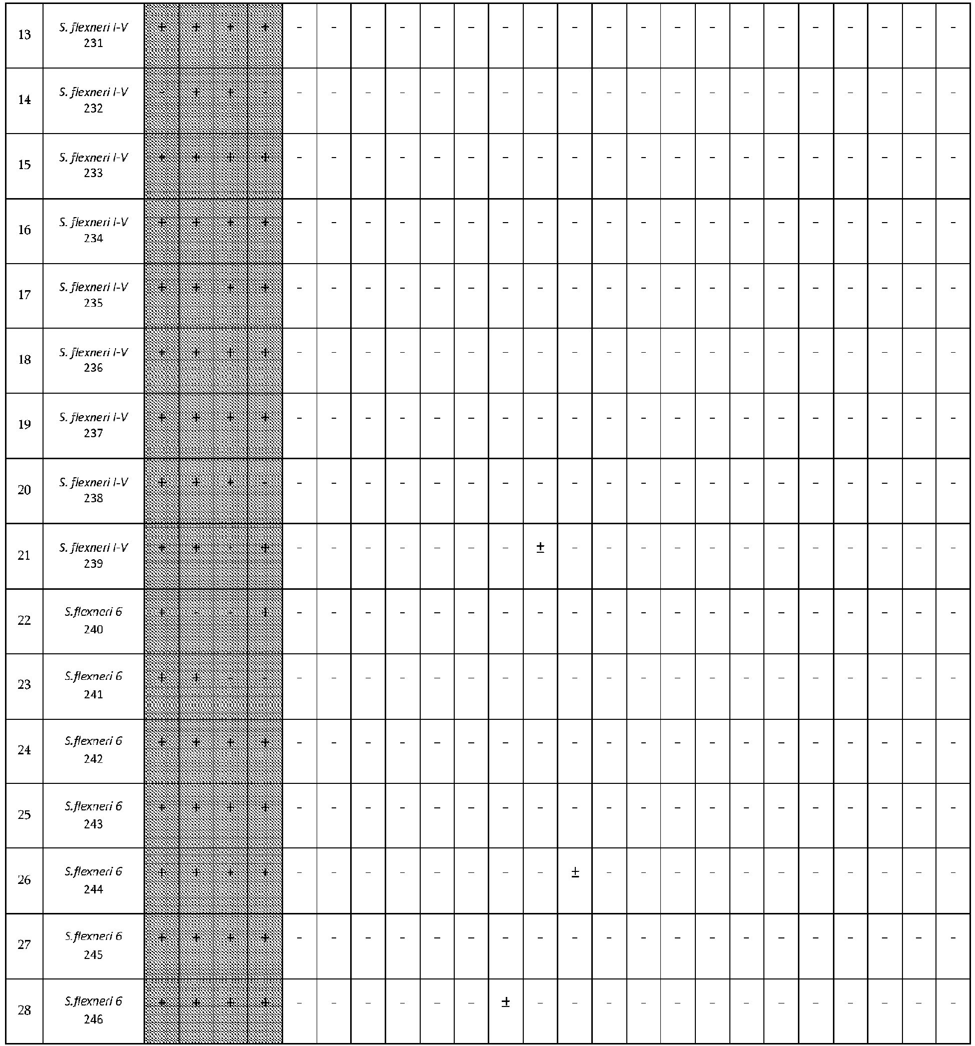

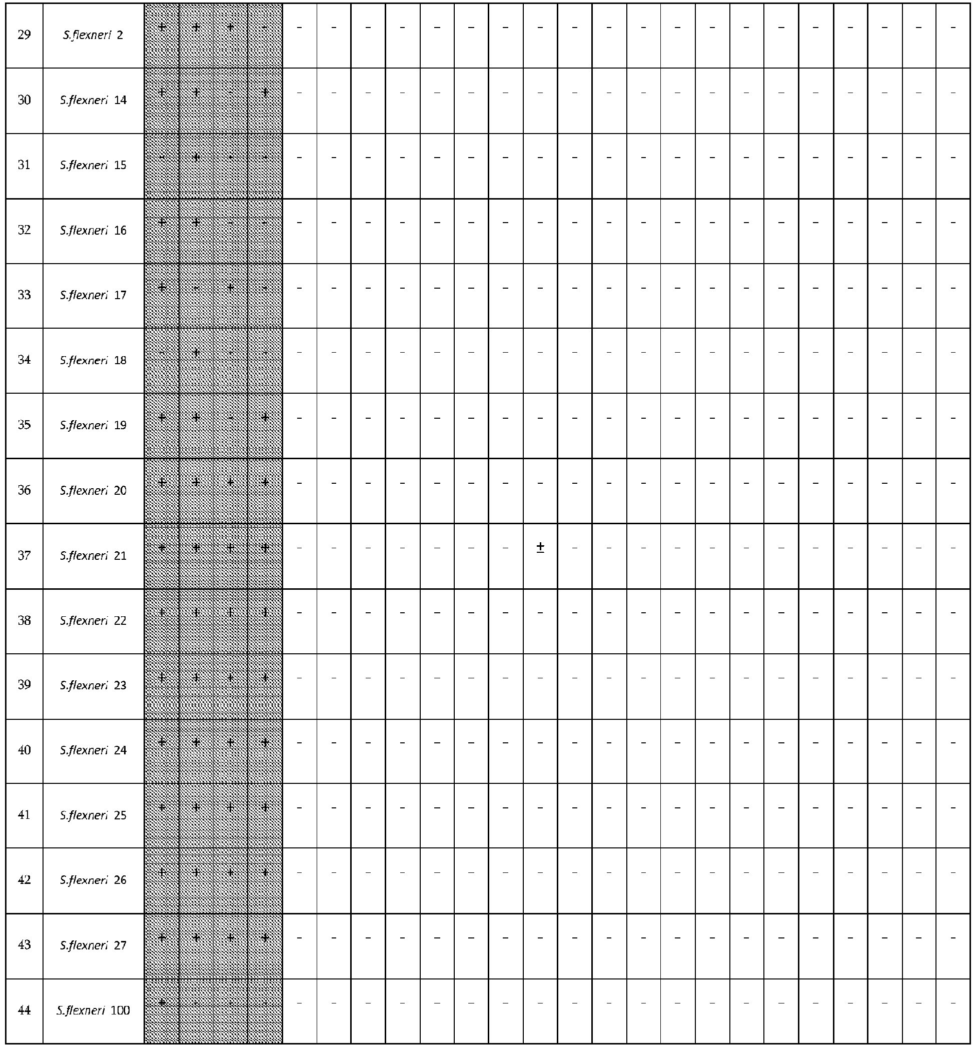

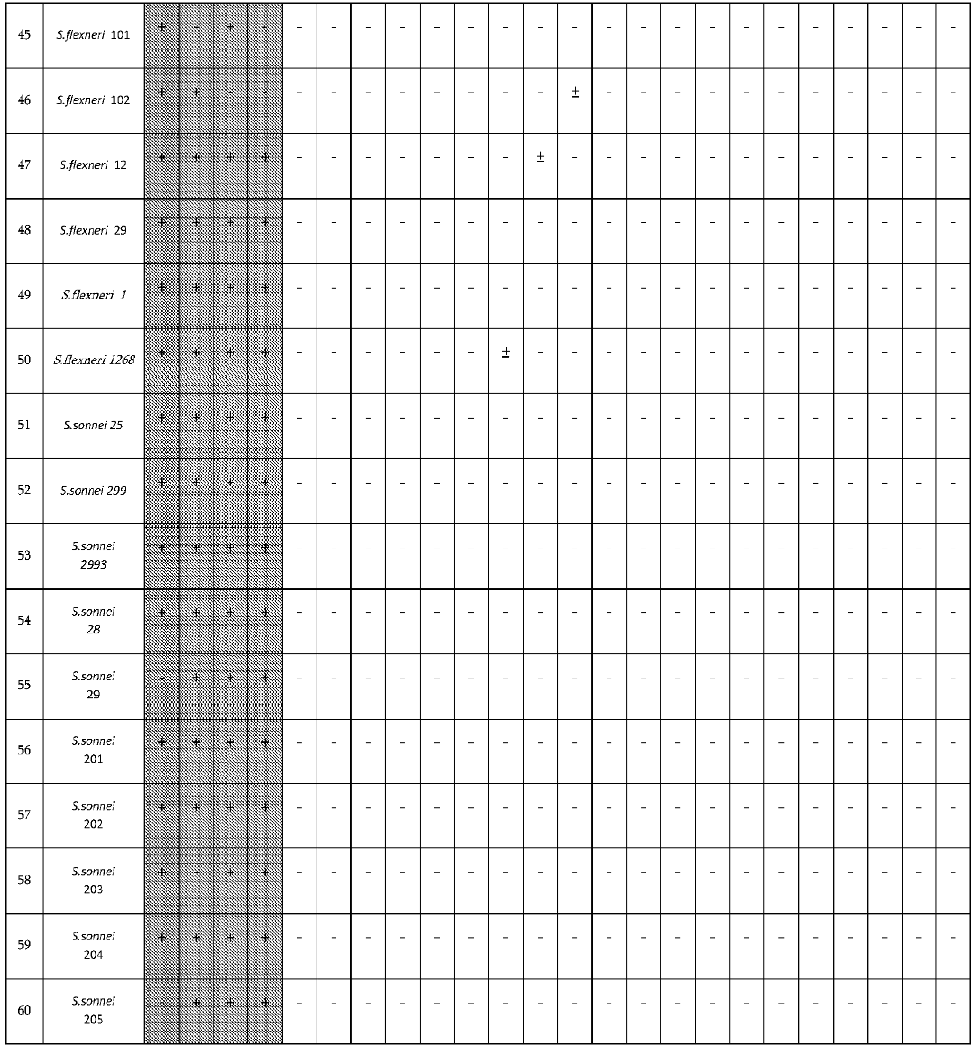

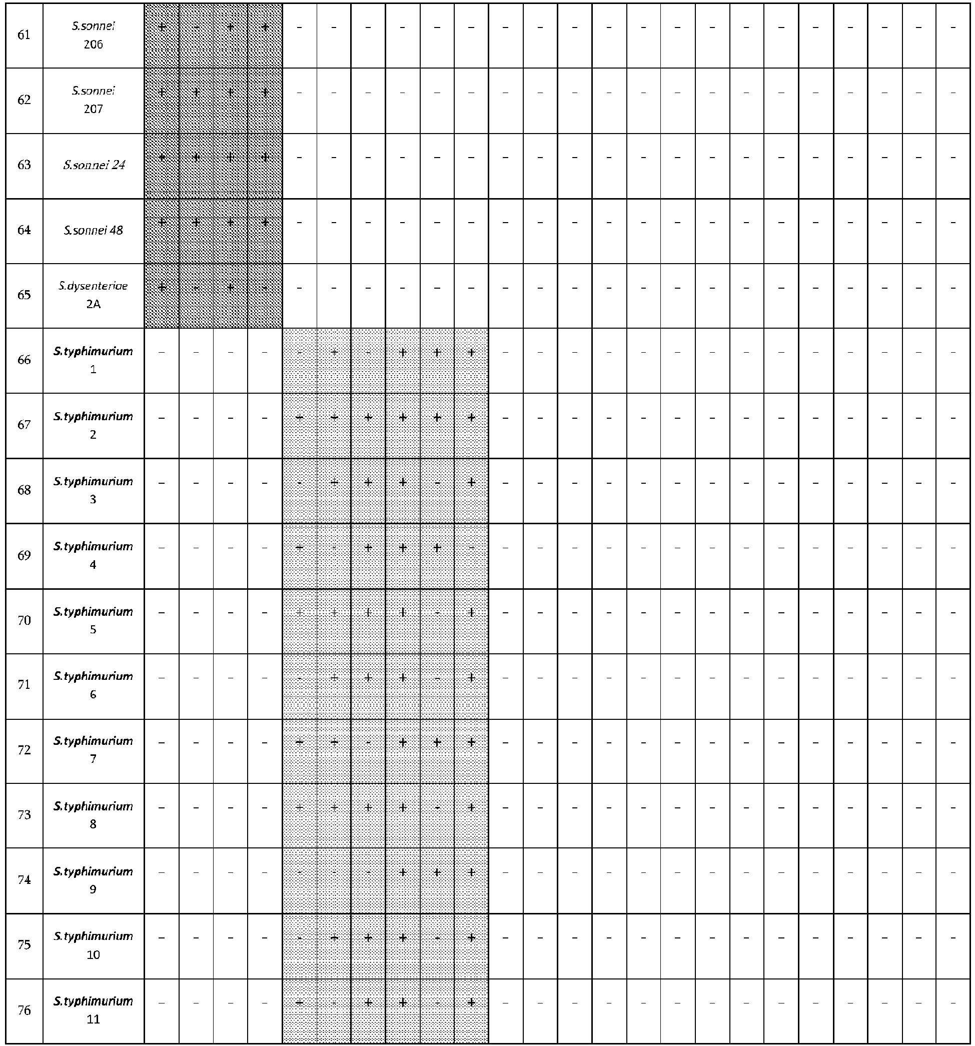

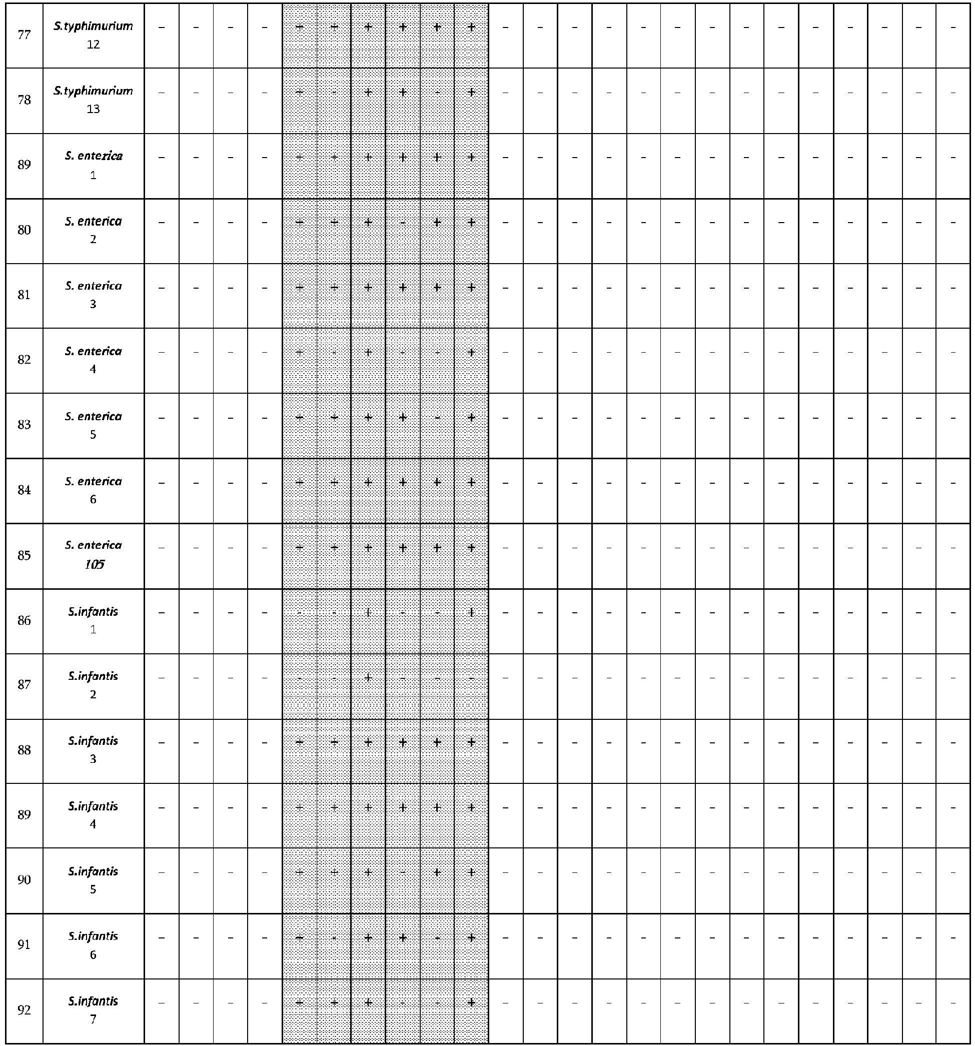

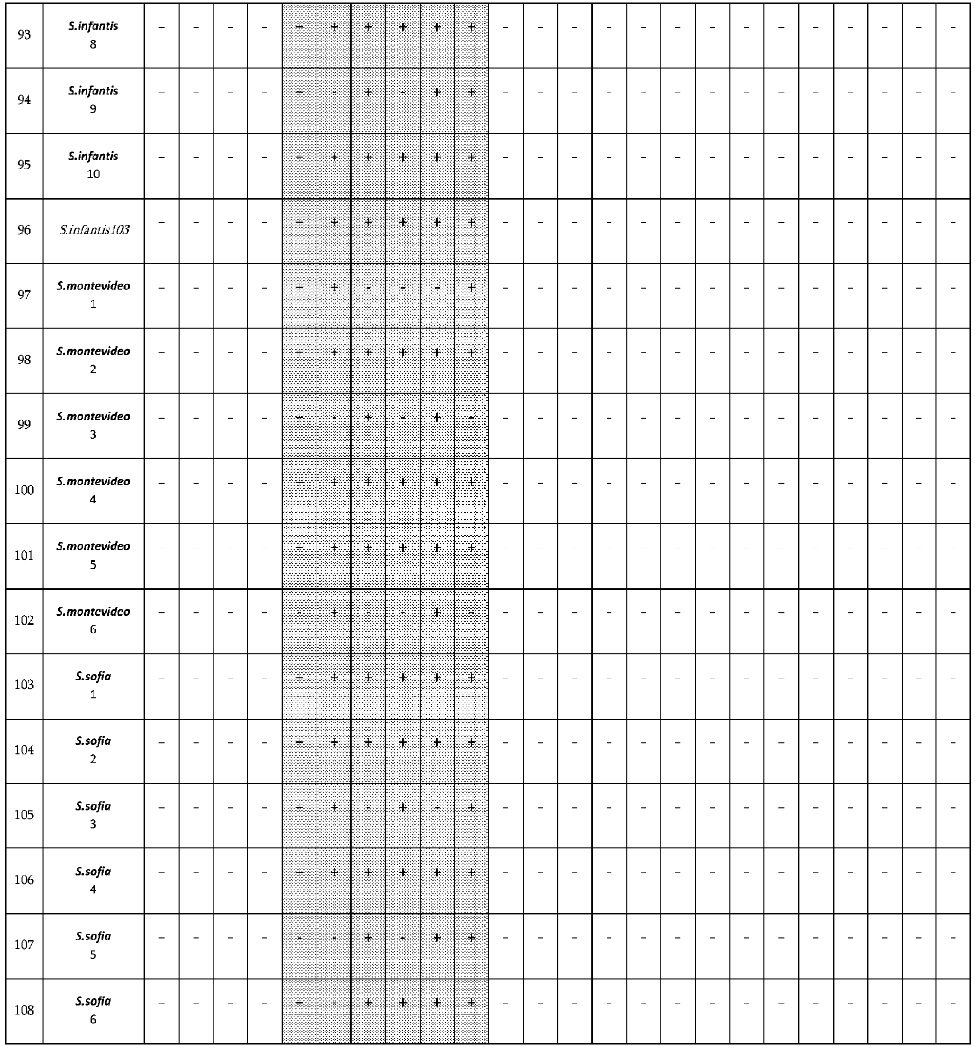

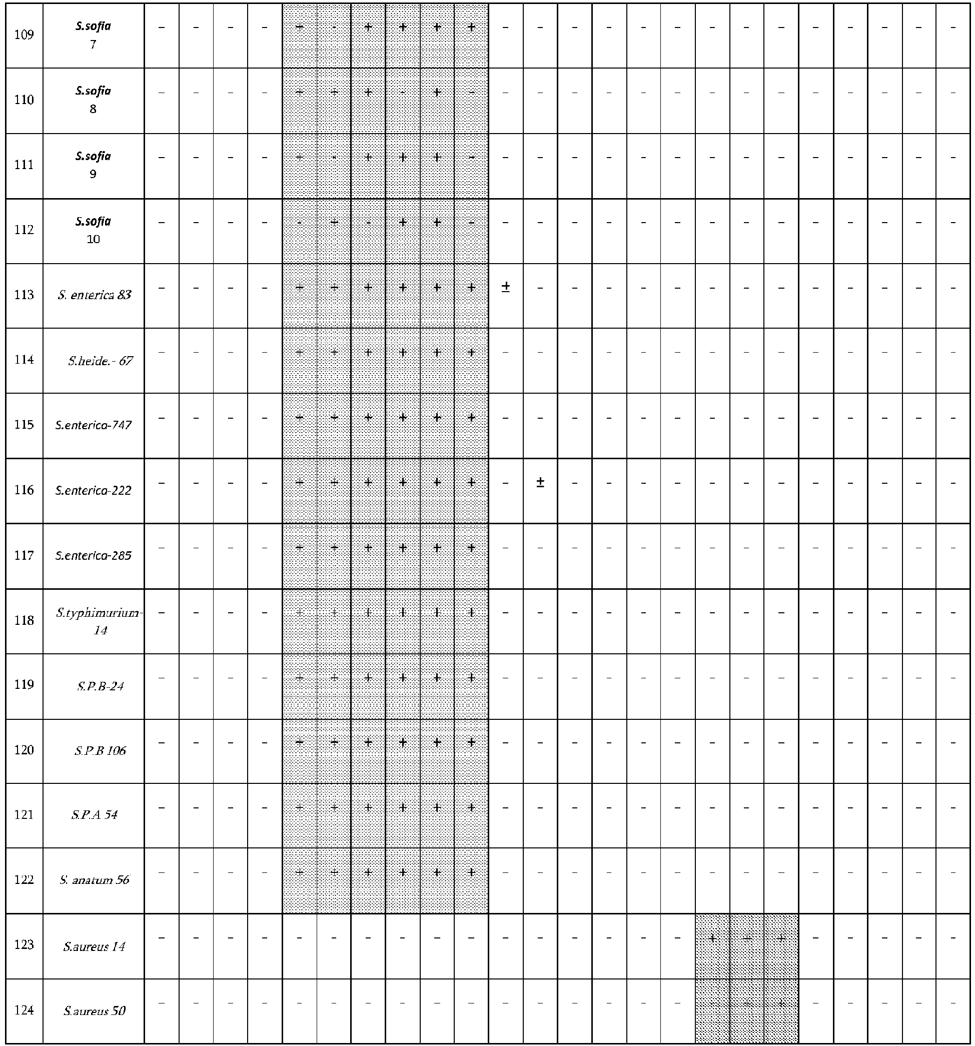

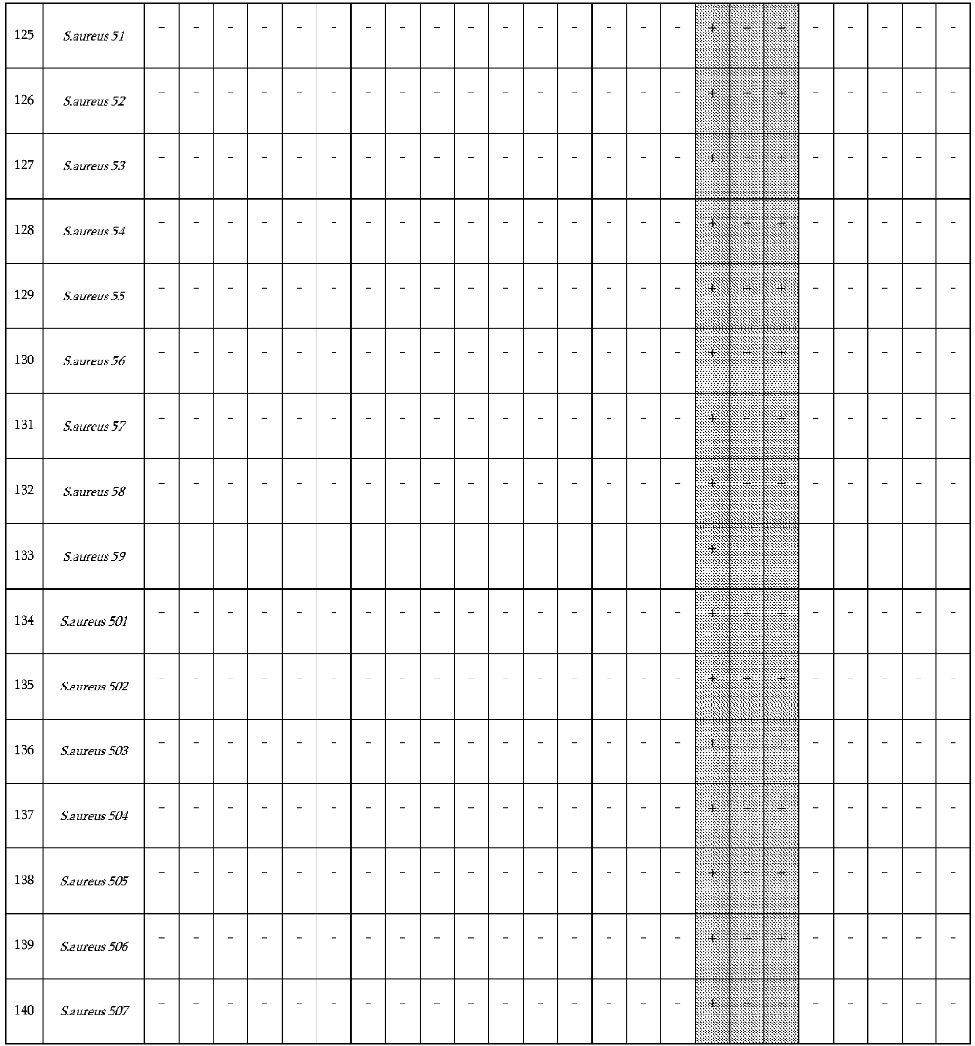

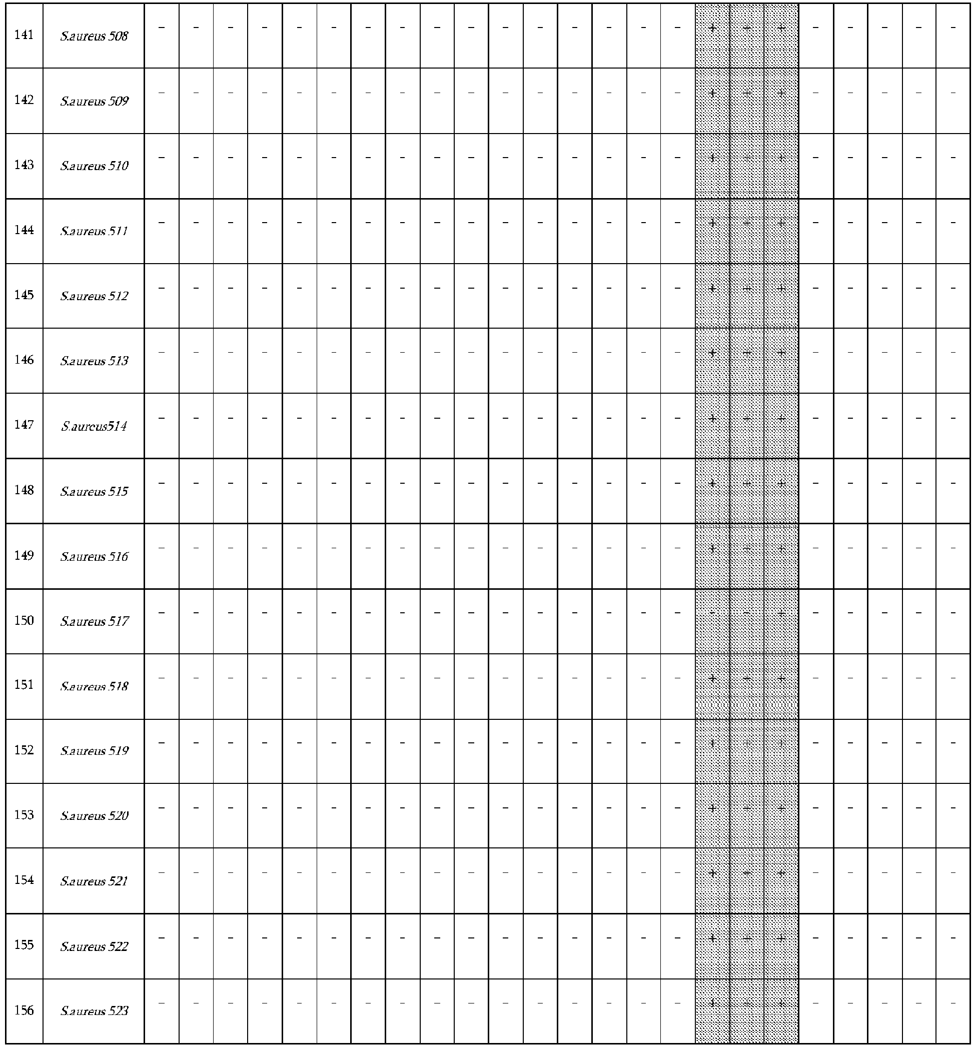

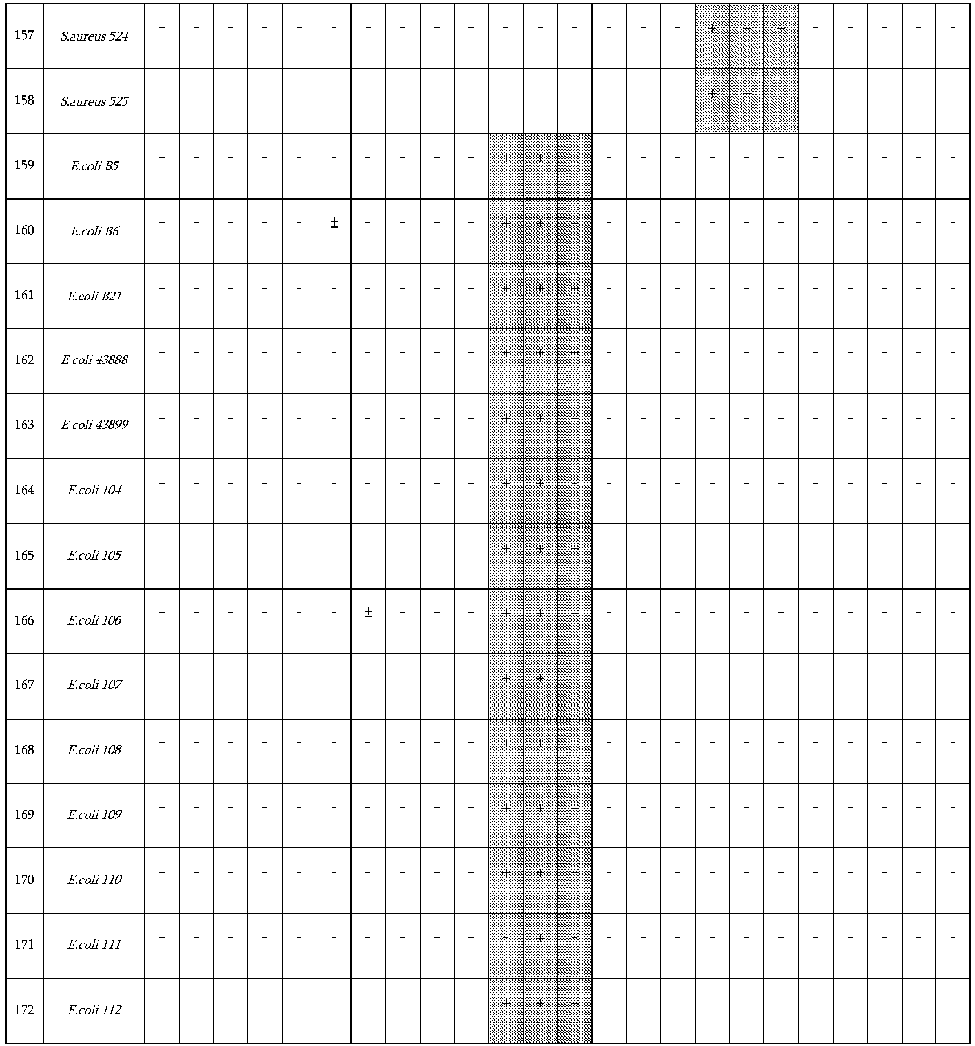

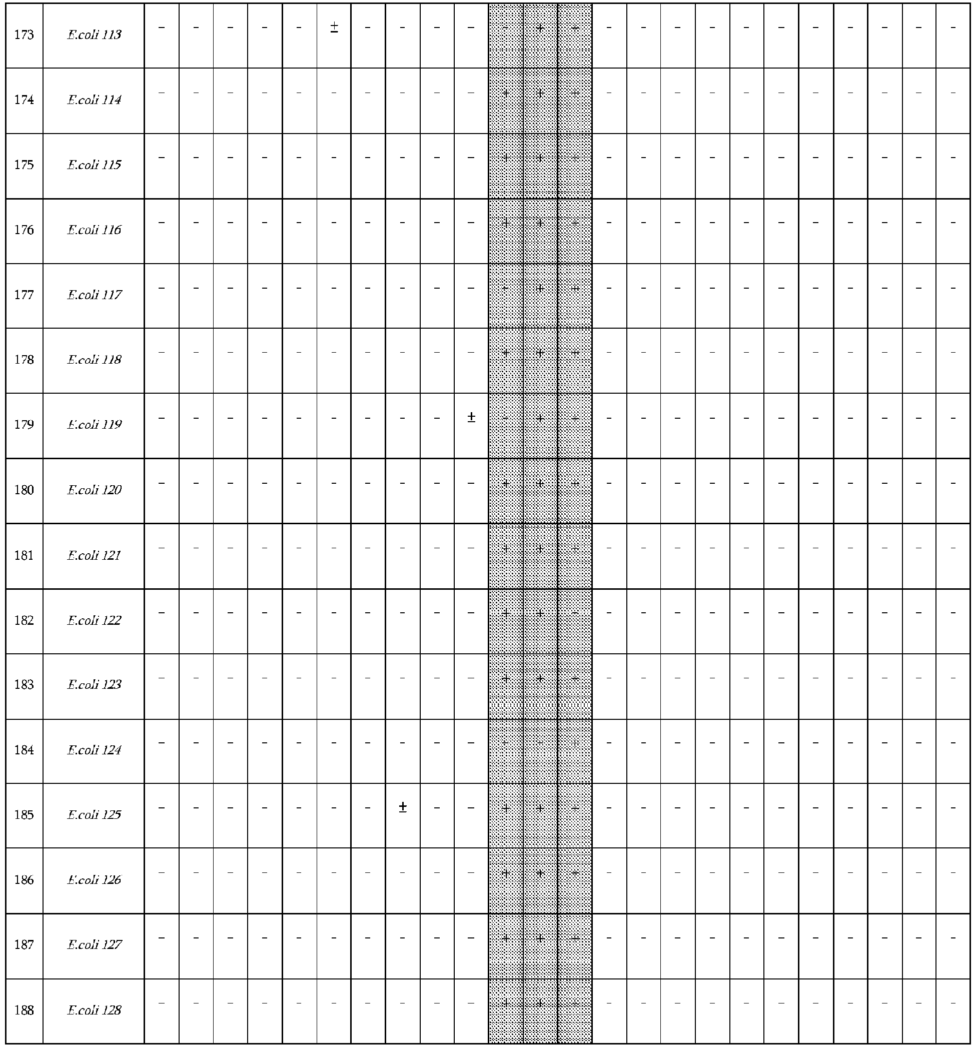

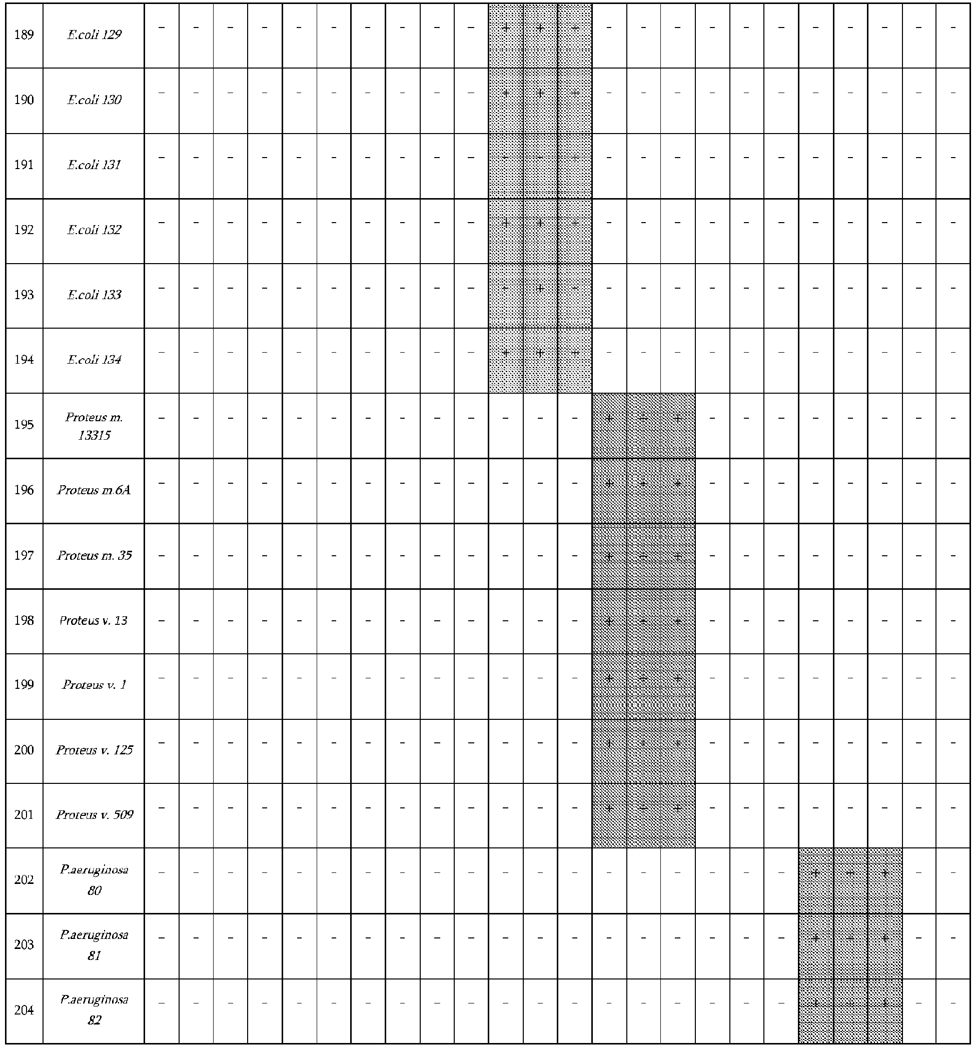

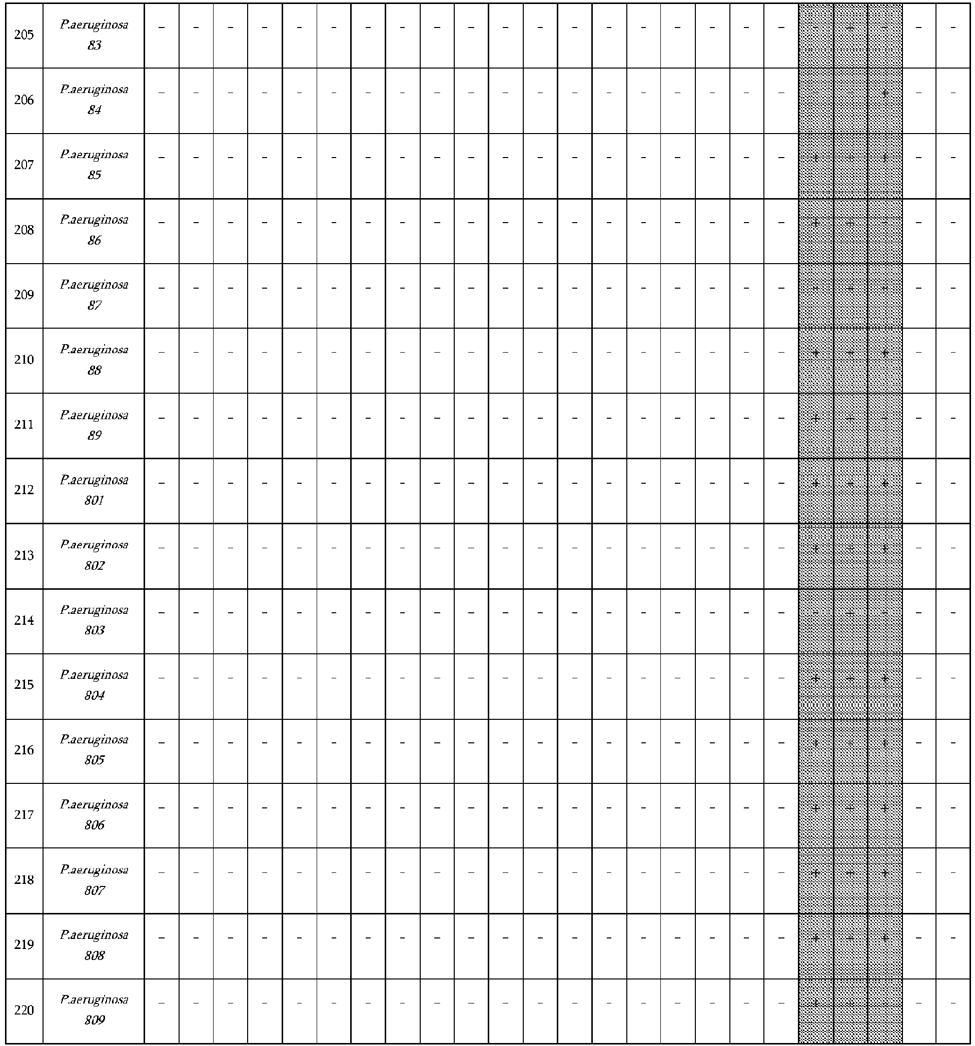

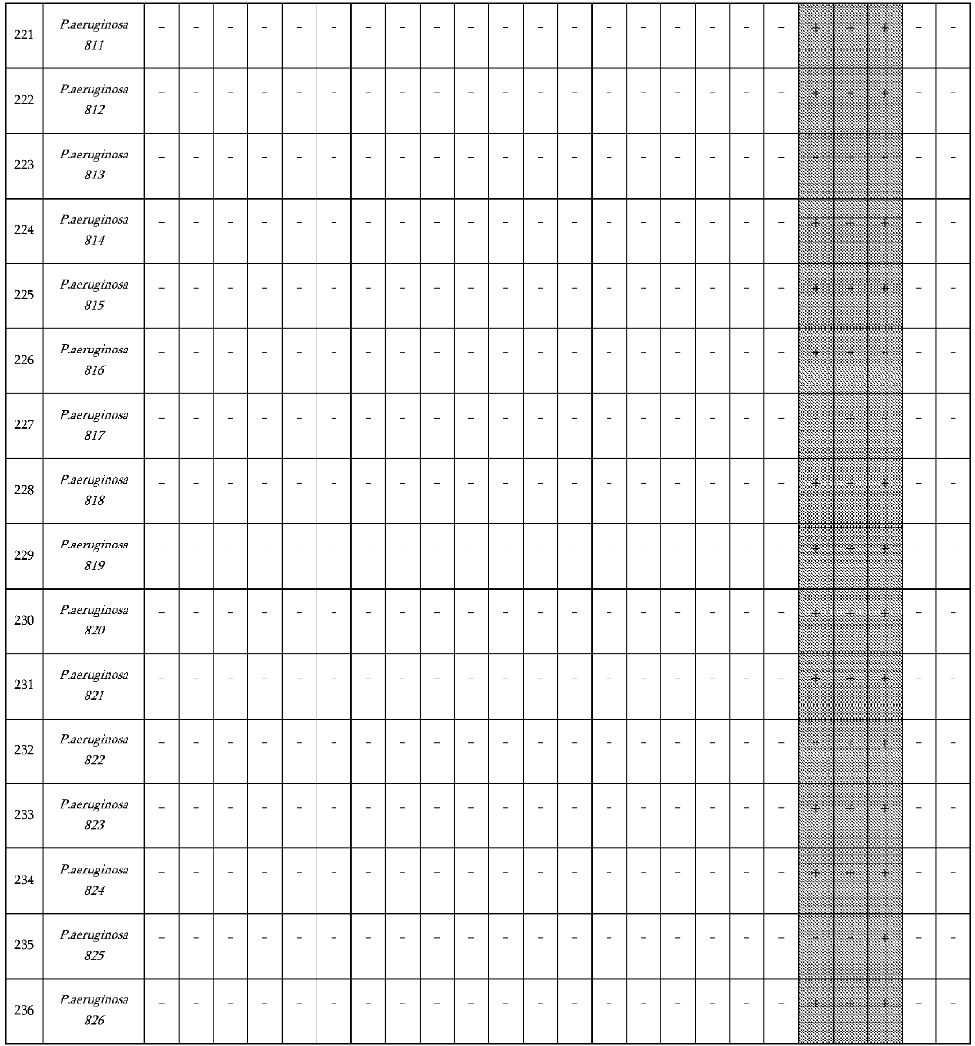

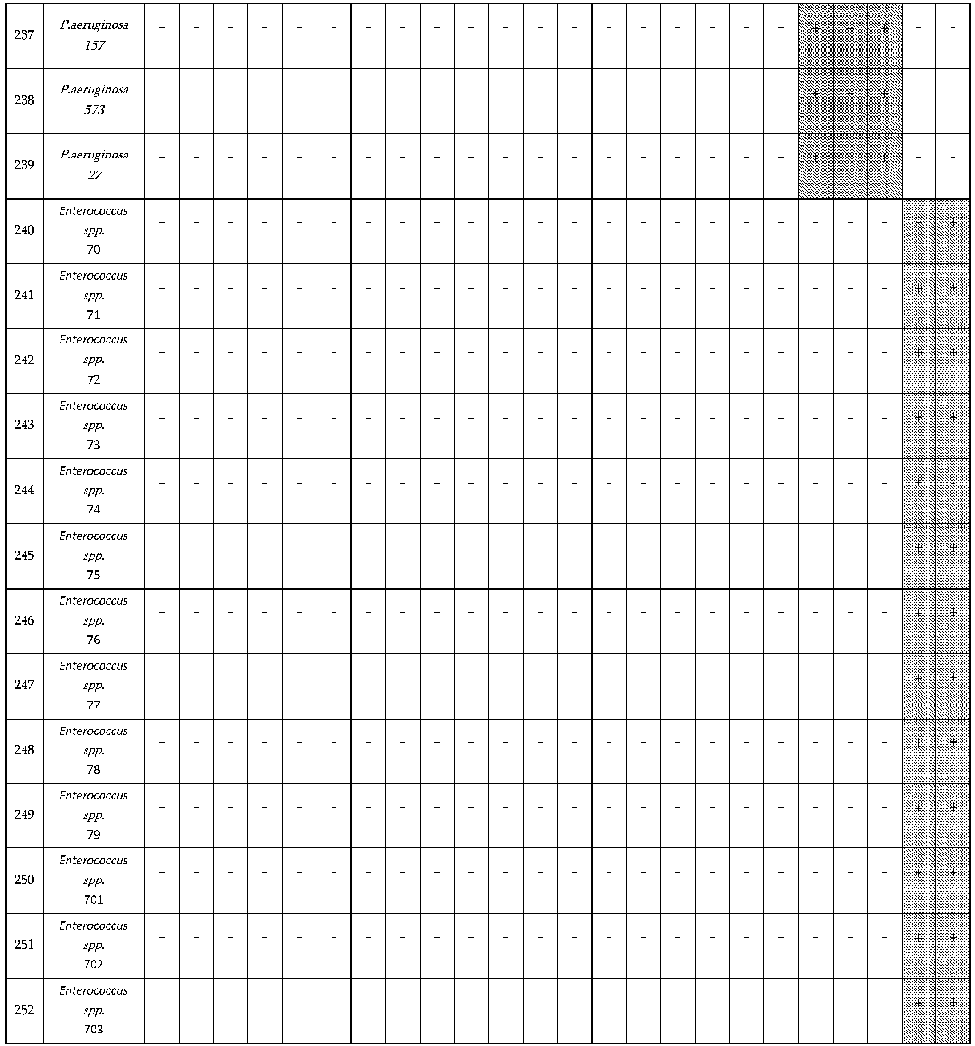

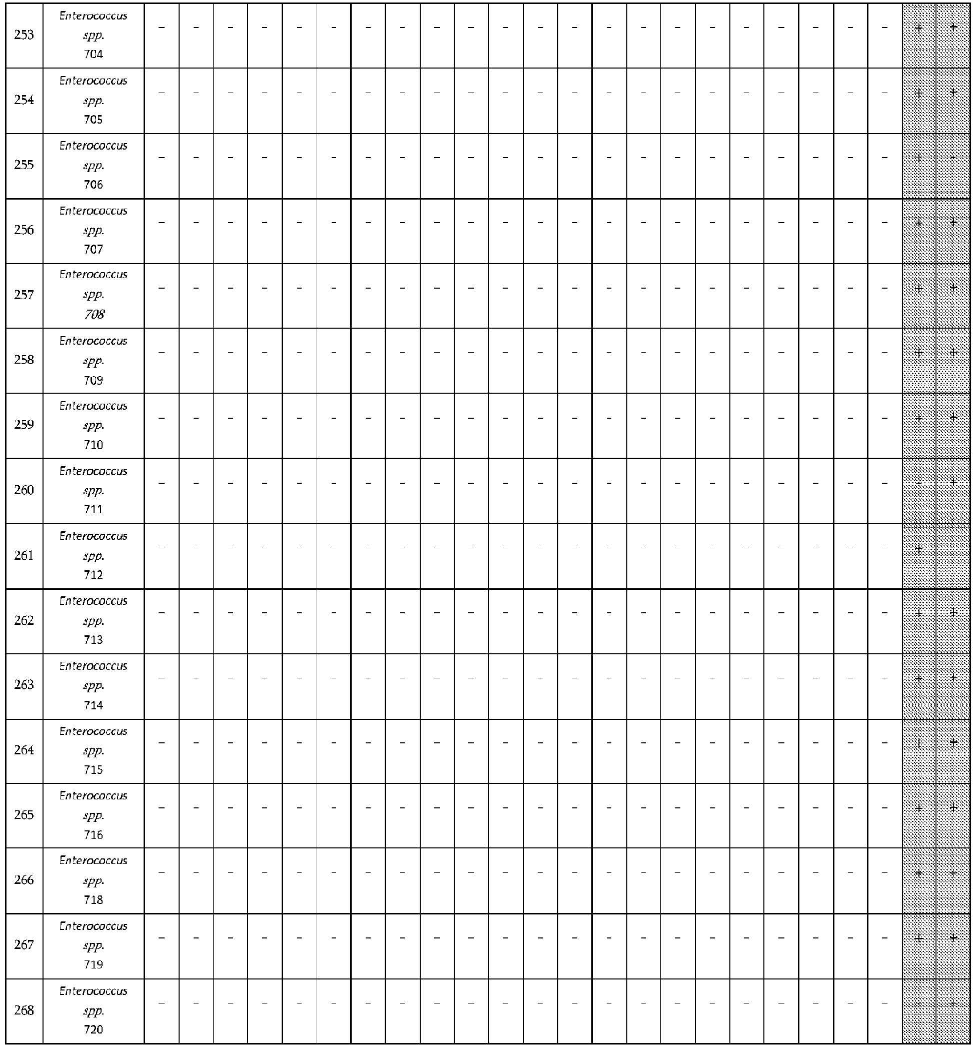

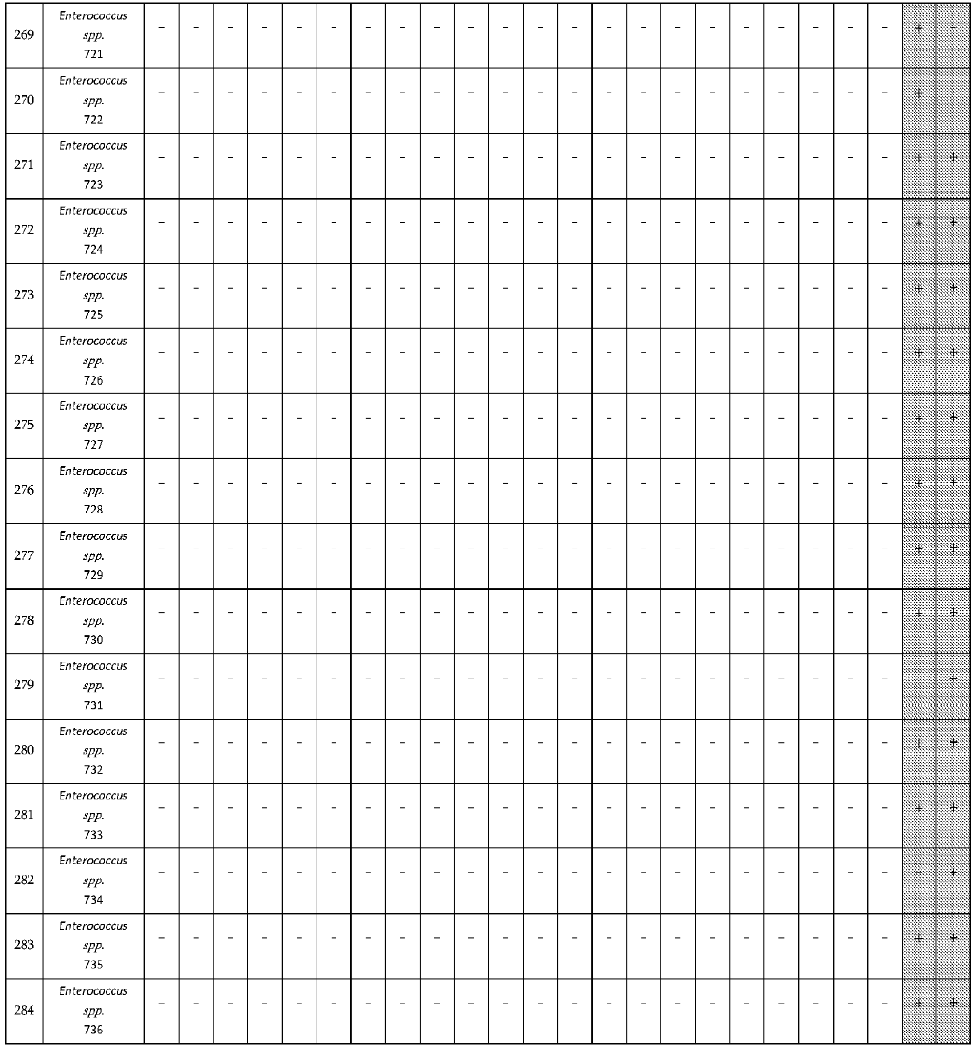

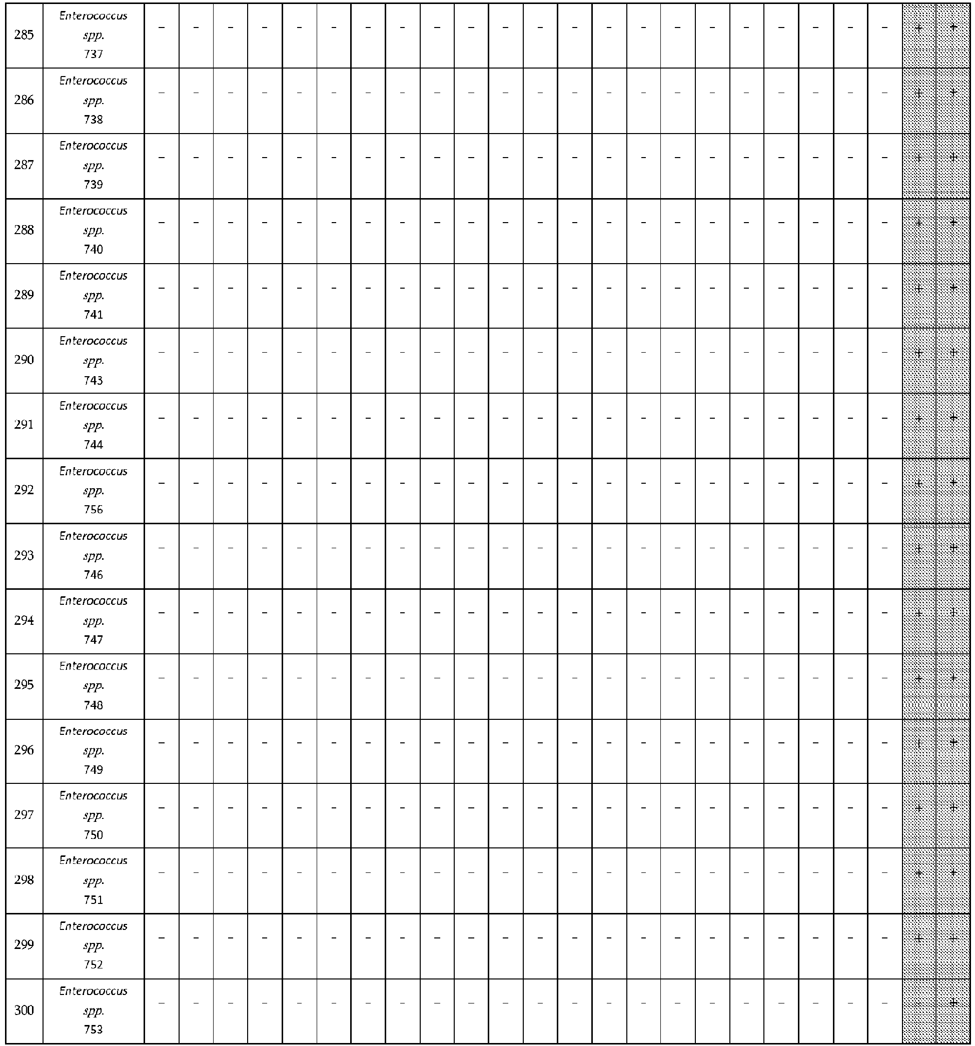

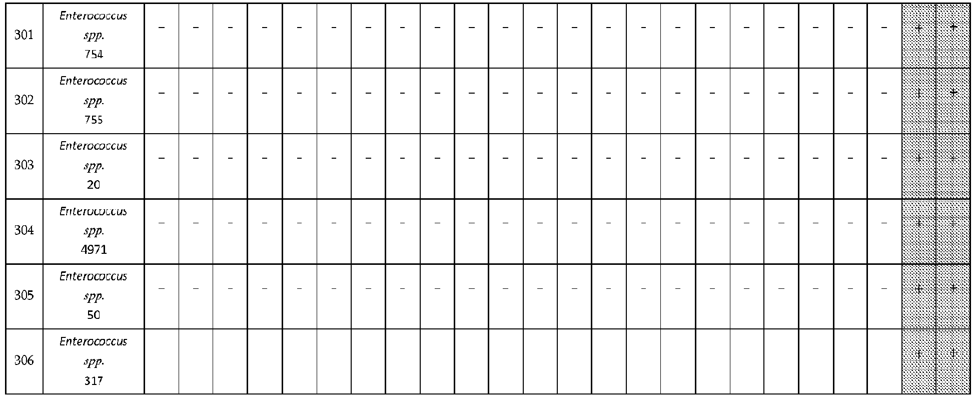

## Claims

1. The antimicrobial composition comprising:
a) Bacteriophage strains, specific to Shigella flexneri. DSM 32619 and DSM 32620, which are deposited in German Collection of Microorganisms and Cell Cultures (DSMZ),
b) Bacteriophage strains, specific to Shigella sonnei. DSM 32621 and DSM 32622, which are deposited in German Collection of Microorganisms and Cell Cultures (DSMZ),
c) Bacteriophage strain, specific to Salmonella choleraesuis. DSM 32625, which is deposited in German Collection of Microorganisms and Cell Cultures (DSMZ),
d) Bacteriophage strain, specific to Salmonella newport DSM 32624, which is deposited in German Collection of Microorganisms and Cell Cultures (DSMZ),
e) Bacteriophage strain, specific to Salmonella paratyphi A: DSM 32623, which is deposited in German Collection of Microorganisms and Cell Cultures (DSMZ),
f) Bacteriophage strain, specific to Salmonella typhimunum. DSM 32626, which is deposited in German Collection of Microorganisms and Cell Cultures (DSMZ),
g) Bacteriophage strain, specific to Salmonella paratyphi B: DSM 32627, which is deposited in German Collection of Microorganisms and Cell Cultures (DSMZ),
h) Bacteriophage strain, specific to Salmonella heidelberg DSM 32628, which is deposited in German Collection of Microorganisms and Cell Cultures (DSMZ),
i) Bacteriophage strains, specific to Escherichia coll. DSM 32612, DSM 32611 and DSM 32610, which are deposited in German Collection of Microorganisms and Cell Cultures (DSMZ),
j) Bacteriophage strains, specific to Proteus vulgaris. DSM 32613, DSM 32614 and DSM 32615, which are deposited in German Collection of Microorganisms and Cell Cultures (DSMZ),
k) Bacteriophage strains, specific to Staphylococcus aureus. DSM 32631, DSM 32629 and DSM 32630, which are deposited in German Collection of Microorganisms and Cell Cultures (DSMZ),
l) Bacteriophage strains, specific to Pseudomonas aeruginosa:. DSM 32616, DSM 32618 and DSM 32617, which are deposited in German Collection of Microorganisms and Cell Cultures (DSMZ),
m) Bacteriophage strains, specific to Enterococcus. DSM 32632 and DSM 32633, which are deposited in German Collection of Microorganisms and Cell Cultures (DSMZ)
and optionally a pharmaceutically acceptable excipient.

2. The composition according to claim 1, wherein the form of the composition is selected from the following group: liquid formulation, spray, tablet, powder, capsule, cream, and suppository.

3. The composition, according to claims 1-2, for use in a treatment and/or prevention of intestinal infections.

4. The composition for use according to claim 3 for use in a treatment and/or prevention of intestinal infections in humans, or in animals and poultry.

5. The composition for use according to claims 3-4, wherein intestinal infections are selected from the following group: dysentery (shigellosis), salmonellosis, escherichiosis (E.coli infection), dyspepsia, dysbiosis, foodbome toxicoinfections, enteritis, gastroenteritis, colitis, enterocolitis and gastroenterocolitis.

6. An isolated bacteriophage strain, with lytic activity against Shigella flexneri is selected from the following group: DSM 32619 and DSM 32620, moreover, the above-mentioned strains are deposited in German Collection of Microorganisms and Cell Cultures (DSMZ).

7. An isolated bacteriophage strain, with lytic activity against Shigella sonnei, is selected from the following group: DSM 32621 and DSM 32622, moreover, the above-mentioned strains are deposited in German Collection of Microorganisms and Cell Cultures (DSMZ).

8. An isolated bacteriophage strain DSM 32625, with lytic activity against Salmonella choleraesuis, which is deposited in German Collection of Microorganisms and Cell Cultures (DSMZ).

9. An isolated bacteriophage strain DSM 32624, with lytic activity against Salmonella newport, which is deposited in German Collection of Microorganisms and Cell Cultures (DSMZ).

10. An isolated bacteriophage strain DSM 32623, with lytic activity against Salmonella paratyphi A, which is deposited in German Collection of Microorganisms and Cell Cultures (DSMZ).

11. An isolated bacteriophage strain DSM 32626, with lytic activity against Salmonella typhimunum, which is deposited in German Collection of Microorganisms and Cell Cultures (DSMZ).

12. An isolated bacteriophage strain DSM 32627, with lytic activity against Salmonella paratyphi B, which is deposited in German Collection of Microorganisms and Cell Cultures (DSMZ).

13. An isolated bacteriophage strain DSM 32628, with lytic activity against Salmonella heidelberg, which is deposited in German Collection of Microorganisms and Cell Cultures (DSMZ).

14. An isolated bacteriophage strain, with lytic activity against Enterococcus, is selected from the following group: DSM 32632 and DSM 32633, moreover, the above-mentioned strains are deposited in German Collection of Microorganisms and Cell Cultures (DSMZ).

## Patentansprüche

1. Antimikrobielle Zusammensetzung, umfassend:
a) Bakteriophagenstämme, die für Shigella flexneri spezifisch sind. DSM 32619 und DSM 32620, die bei der Deutschen Sammlung von Mikroorganismen und Zellkulturen (DSMZ) hinterlegt sind,
b) Bakteriophagenstämme, die für Shigella sonnei spezifisch sind. DSM 32621 und DSM 32622, die bei der Deutschen Sammlung von Mikroorganismen und Zellkulturen (DSMZ) hinterlegt sind,
c) Bakteriophagenstamm, der für Salmonella choleraesuis spezifisch ist. DSM 32625, der bei der Deutschen Sammlung von Mikroorganismen und Zellkulturen (DSMZ) hinterlegt ist,
d) Bakteriophagenstamm, der für Salmonella newport spezifisch ist, DSM 32624, der bei der Deutschen Sammlung von Mikroorganismen und Zellkulturen (DSMZ) hinterlegt ist,
e) Bakteriophagenstamm, der für Salmonella paratyphi A spezifisch ist: DSM 32623, der bei der Deutschen Sammlung von Mikroorganismen und Zellkulturen (DSMZ) hinterlegt ist,
f) Bakteriophagenstamm, der für Salmonella typhimunum spezifisch ist. DSM 32626, der bei der Deutschen Sammlung von Mikroorganismen und Zellkulturen (DSMZ) hinterlegt ist,
g) Bakteriophagenstamm, der für Salmonella paratyphi B spezifisch ist: DSM 32627, der bei der Deutschen Sammlung von Mikroorganismen und Zellkulturen (DSMZ) hinterlegt ist,
h) Bakteriophagenstamm, der für Salmonella heidelberg spezifisch ist, DSM 32628, der bei der Deutschen Sammlung von Mikroorganismen und Zellkulturen (DSMZ) hinterlegt ist,
i) Bakteriophagenstämme, die für Escherichia coll. spezifisch sind DSM 32612, DSM 32611 und DSM 32610, die bei der Deutschen Sammlung von Mikroorganismen und Zellkulturen (DSMZ) hinterlegt sind,
j) Bakteriophagenstämme, die für Proteus vulgaris spezifisch sind DSM 32613, DSM 32614 und DSM 32615, die bei der Deutschen Sammlung von Mikroorganismen und Zellkulturen (DSMZ) hinterlegt sind,
k) Bakteriophagenstämme, die für Staphylococcus aureus spezifisch sind DSM 32631, DSM 32629 und DSM 32630, die bei der Deutschen Sammlung von Mikroorganismen und Zellkulturen (DSMZ) hinterlegt sind,
l) Bakteriophagenstämme, die für Pseudomonas aeruginosa spezifisch sind DSM 32616, DSM 32618 und DSM 32617, die bei der Deutschen Sammlung von Mikroorganismen und Zellkulturen (DSMZ) hinterlegt sind,
m) Bakteriophagenstämme, die für Enterococcus spezifisch sind DSM 32632 und DSM 32633, die bei der Deutschen Sammlung von Mikroorganismen und Zellkulturen (DSMZ) hinterlegt sind
und gegebenenfalls einen pharmazeutisch akzeptablen Hilfsstoff.

2. Zusammensetzung nach Anspruch 1, wobei die Form der Zusammensetzung aus der folgenden Gruppe ausgewählt ist: flüssige Formulierung, Spray, Tablette, Pulver, Kapsel, Creme und Zäpfchen.

3. Zusammensetzung nach den Ansprüchen 1-2 zur Verwendung bei einer Behandlung und/oder Prävention von Darminfektionen.

4. Zusammensetzung zur Verwendung nach Anspruch 3 zur Verwendung bei einer Behandlung und/oder Prävention von Darminfektionen bei Menschen oder bei Tieren und Geflügel.

5. Zusammensetzung zur Verwendung nach den Ansprüchen 3-4, wobei die Darminfektionen aus der folgenden Gruppe ausgewählt sind: Dysenterie (Shigellosis), Salmonellose, Escherichiose (E. coli-Infektion), Dyspepsie, Dysbiose, Lebensmittelvergiftungen, Enteritis, Gastroenteritis, Colitis, Enterocolitis und Gastroenterocolitis.

6. Isolierter Bakteriophagenstamm mit lytischer Aktivität gegen Shigella flexneri, der aus der folgenden Gruppe ausgewählt ist: DSM 32619 und DSM 32620, wobei die oben genannten Stämme außerdem in der Deutschen Sammlung von Mikroorganismen und Zellkulturen (DSMZ) hinterlegt sind.

7. Isolierter Bakteriophagenstamm mit lytischer Aktivität gegen Shigella sonnei, der aus der folgenden Gruppe ausgewählt ist: DSM 32621 und DSM 32622, wobei die oben genannten Stämme außerdem in der Deutschen Sammlung von Mikroorganismen und Zellkulturen (DSMZ) hinterlegt sind.

8. Isolierter Bakteriophagenstamm DSM 32625 mit lytischer Aktivität gegen Salmonella choleraesuis, der bei der Deutschen Sammlung von Mikroorganismen und Zellkulturen (DSMZ) hinterlegt ist.

9. Isolierter Bakteriophagenstamm DSM 32624 mit lytischer Aktivität gegen Salmonella newport, der bei der Deutschen Sammlung von Mikroorganismen und Zellkulturen (DSMZ) hinterlegt ist.

10. Isolierter Bakteriophagenstamm DSM 32623 mit lytischer Aktivität gegen Salmonella paratyphi A, der bei der Deutschen Sammlung von Mikroorganismen und Zellkulturen (DSMZ) hinterlegt ist.

11. Isolierter Bakteriophagenstamm DSM 32626 mit lytischer Aktivität gegen Salmonella typhimunum, der bei der Deutschen Sammlung von Mikroorganismen und Zellkulturen (DSMZ) hinterlegt ist.

12. Isolierter Bakteriophagenstamm DSM 32627 mit lytischer Aktivität gegen Salmonella paratyphi B, der bei der Deutschen Sammlung von Mikroorganismen und Zellkulturen (DSMZ) hinterlegt ist.

13. Isolierter Bakteriophagenstamm DSM 32628 mit lytischer Aktivität gegen Salmonella heidelberg, der bei der Deutschen Sammlung von Mikroorganismen und Zellkulturen (DSMZ) hinterlegt ist.

14. Isolierter Bakteriophagenstamm mit lytischer Aktivität gegen Enterococcus, der aus der folgenden Gruppe ausgewählt ist: DSM 32632 und DSM 32633, wobei die oben genannten Stämme außerdem in der Deutschen Sammlung von Mikroorganismen und Zellkulturen (DSMZ) hinterlegt sind.

## Revendications

1. Composition antimicrobienne comprenant :
a) des souches de bactériophages, spécifiques pour Shigella flexneri. DSM 32619 et DSM 32620, qui sont déposées à la Collection allemande de microorganismes et de cultures cellulaires (DSMZ),
b) des souches de bactériophages, spécifiques pour Shigella sonnei. DSM 32621 et DSM 32622, qui sont déposées à la Collection allemande de microorganismes et de cultures cellulaires (DSMZ),
c) une souche de bactériophages, spécifique pour Salmonella choleraesuis. DSM 32625, qui est déposée à la Collection allemande de microorganismes et de cultures cellulaires (DSMZ),
d) une souche de bactériophages, spécifique pour Salmonella newport DSM 32624, qui est déposée à la Collection allemande de microorganismes et de cultures cellulaires (DSMZ),
e) une souche de bactériophages, spécifique pour Salmonella paratyphi A : DSM 32623, qui est déposée à la Collection allemande de microorganismes et de cultures cellulaires (DSMZ),
f) une souche de bactériophages, spécifique pour Salmonella typhimunum. DSM 32626, qui est déposée à la Collection allemande de microorganismes et de cultures cellulaires (DSMZ),
g) une souche de bactériophages, spécifique pour Salmonella paratyphi B : DSM 32627, qui est déposée à la Collection allemande de microorganismes et de cultures cellulaires (DSMZ),
h) une souche de bactériophages, spécifique pour Salmonella heidelberg DSM 32628, qui est déposée à la Collection allemande de microorganismes et de cultures cellulaires (DSMZ),
i) des souches de bactériophages, spécifiques pour Escherichia coll. DSM 32612, DSM 32611 et DSM 32610, qui sont déposées à la Collection allemande de microorganismes et de cultures cellulaires (DSMZ),
j) des souches de bactériophages, spécifiques pour Proteus vulgaris. DSM 32613, DSM 32614 et DSM 32615, qui sont déposées à la Collection allemande de microorganismes et de cultures cellulaires (DSMZ),
k) des souches de bactériophages, spécifiques pour Staphylococcus aureus. DSM 32631, DSM 32629 et DSM 32630, qui sont déposées à la Collection allemande de microorganismes et de cultures cellulaires (DSMZ),
l) des souches de bactériophages, spécifiques pour Pseudomonas aeruginosa. DSM 32616, DSM 32618 et DSM 32617, qui sont déposées à la Collection allemande de microorganismes et de cultures cellulaires (DSMZ),
m) des souches de bactériophages, spécifiques pour Enterococcus. DSM 32632 et DSM 32633, qui sont déposées à la Collection allemande de microorganismes et de cultures cellulaires (DSMZ),
et éventuellement un excipient pharmaceutiquement acceptable.

2. Composition selon la revendication 1, dans laquelle la forme de la composition est choisie dans le groupe suivant : formulation liquide, spray, comprimé, poudre, capsule, crème et suppositoire.

3. Composition, selon les revendications 1 à 2, pour une utilisation dans un traitement et/ou une prévention d'infections intestinales.

4. Composition pour une utilisation selon la revendication 3, pour une utilisation dans un traitement et/ou une prévention d'infections intestinales chez l'homme, ou chez les animaux et la volaille.

5. Composition pour une utilisation selon les revendications 3 à 4, dans laquelle les infections intestinales sont choisies dans le groupe suivant : dysenterie (shigellose), salmonellose, eschéichiose (infection par E. coli), dyspepsie, dysbiose, toxicoinfections alimentaires, entérite, gastro-entérite, colite, entérocolite et gastro-entérocolite.

6. Souche de bactériophages isolée, ayant une activité lytique contre Shigella flexneri, choisie dans le groupe suivant : DSM 32619 et DSM 32620, de plus, les souches susmentionnées sont déposées dans la Collection allemande de microorganismes et de cultures cellulaires (DSMZ).

7. Souche de bactériophages isolée, ayant une activité lytique contre Shigella sonnei, choisie dans le groupe suivant : DSM 32621 et DSM 32622, de plus, les souches susmentionnées sont déposées dans la Collection allemande de microorganismes et de cultures cellulaires (DSMZ).

8. Souche de bactériophages isolée DSM 32625, ayant une activité lytique contre Salmonella choleraesuis, qui est déposée à la Collection allemande de microorganismes et de cultures cellulaires (DSMZ).

9. Souche de bactériophages isolée DSM 32624, ayant une activité lytique contre Salmonella newport, qui est déposée à la Collection allemande de microorganismes et de cultures cellulaires (DSMZ).

10. Souche de bactériophages isolée DSM 32623, ayant une activité lytique contre Salmonella paratyphi A, qui est déposée à la Collection allemande de microorganismes et de cultures cellulaires (DSMZ).

11. Souche de bactériophages isolée DSM 32626, ayant une activité lytique contre Salmonella typhimunum, qui est déposée à la Collection allemande de microorganismes et de cultures cellulaires (DSMZ).

12. Souche de bactériophages isolée DSM 32627, ayant une activité lytique contre Salmonella paratyphi B, qui est déposée à la Collection allemande de microorganismes et de cultures cellulaires (DSMZ).

13. Souche de bactériophages isolée DSM 32628, ayant une activité lytique contre Salmonella heidelberg, qui est déposée à la Collection allemande de microorganismes et de cultures cellulaires (DSMZ).

14. Souche de bactériophages isolée, ayant une activité lytique contre Enterococcus, choisie dans le groupe suivant : DSM 32632 et DSM 32633, de plus, les souches susmentionnées sont déposées dans la Collection allemande de microorganismes et de cultures cellulaires (DSMZ).
